(19) Europäisches Patentamt / European Patent Office / Office européen des brevets

(11) **EP 4 352 258 B1**

(12) **EUROPEAN PATENT SPECIFICATION**

(45) Date of publication and mention of the grant of the patent:
**29.07.2026 Bulletin 2026/31**

(21) Application number: **22821002.7**

(22) Date of filing: **09.06.2022**

(51) International Patent Classification (IPC):
***C12Q 1/6851*** (2018.01)    ***C12Q 1/70*** (2006.01)
***C12Q 1/6876*** (2018.01)

(52) Cooperative Patent Classification (CPC):
(C-Sets available)
**C12Q 1/708; C12Q 1/686; C12Q 1/6886;**
C12Q 2600/158    (Cont.)

(86) International application number:
**PCT/US2022/032766**

(87) International publication number:
**WO 2022/261274 (15.12.2022 Gazette 2022/50)**

(54) **MATERIALS AND METHODS FOR MEASURING HPV CTDNA**

MATERIALIEN UND VERFAHREN ZUR MESSUNG VON HPV-CTDNA

SUBSTANCES ET MÉTHODES DE MESURE D'ADNTC DE VPH

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.06.2021 US 202163208736 P**

(43) Date of publication of application:
**17.04.2024 Bulletin 2024/16**

(73) Proprietor: **The Regents of the University of Michigan**
Ann Arbor, Michigan 48109-1280 (US)

(72) Inventors:
• **BRENNER, Chad**
  Ann Arbor, Michigan 48109 (US)
• **TEWARI, Muneesh**
  Ann Arbor, Michigan 48109 (US)
• **SWIECICKI, Paul**
  Ann Arbor, Michigan 48109 (US)
• **BHAMBHANI, Chandan**
  Ann Arbor, Michigan 48109 (US)

(74) Representative: **GLAWE DELFS MOLL**
**Partnerschaft mbB**
**Hopfenmarkt 33**
**20457 Hamburg (DE)**

(56) References cited:
WO-A1-2016/209703     WO-A1-2020/060994
WO-A1-2020/160502     WO-A1-2021/046655
WO-A2-2020/055834     US-A1- 2003 124 530
US-A1- 2009 247 607

• **HANNA G.J. ET AL: "Plasma HPV cell-free DNA monitoring in advanced HPV-associated oropharyngeal cancer", vol. 29, no. 9, 13 July 2018 (2018-07-13), pages 1980 - 1986, XP055963249, Retrieved from the Internet <URL:https://www.annalsofoncology.org/action/showPdf?pii=S0923-7534(19)34172-9> DOI: 10.1093/annonc/mdy251**
• **AHN ET AL.: "Saliva and plasma quantitative polymerase chain reaction-based detection and surveillance of human papillomavirus-related head and neck cancer", JAMA OTOLARYNGOL HEAD NECK SURG, vol. 140, no. 9, September 2014 (2014-09-01), pages 846 - 854, XP055341074, DOI: 10.1001/jamaoto.2014.1338**

• CHERA BHISHAMJIT S., KUMAR SUNIL, BEATY BRIAN T., MARRON DAVID, JEFFERYS STUART, GREEN REBECCA, GOLDMAN EMILY C., AMDUR ROBERT, SH: "Rapid Clearance Profile of Plasma Circulating Tumor HPV Type 16 DNA during Chemoradiotherapy Correlates with Disease Control in HPV-Associated Oropharyngeal Cancer", CLINICAL CANCER RESEARCH, ASSOCIATION FOR CANCER RESEARCH, US, vol. 25, no. 15, 1 August 2019 (2019-08-01), US, pages 4682 - 4690, XP093018718, ISSN: 1078-0432, DOI: 10.1158/1078-0432.CCR-19-0211

Remarks:
The complete document including Reference Table(s) and the Sequence Listing(s) can be downloaded from the EPO website

(52) Cooperative Patent Classification (CPC): (Cont.)

C-Sets
**C12Q 1/686, C12Q 2563/159, C12Q 2561/101**

Remarks:

**Description**

**STATEMENT OF RELATED APPLICATIONS**

This application claims priority to U.S. Provisional Patent Application No. 63/208,736, filed June 9, 2021.

**SEQUENCE LISTING**

The text of the computer readable sequence listing filed herewith, titled "2024-03-12_39554-611_SQLnew_ST25", createdMarch 12, 2024, having a file size of 3,133 bytes, is provided.

**FIELD**

The present disclosure relates to materials and methods for measuring human papillomavirus (HPV) circulating tumor DNA (ctDNA). In particular, the disclosure provides a droplet digital PCR-based assay for HPV16 ctDNA, and the use thereof for predicting response to treatment in patients having HPV positive head and neck squamous cell carcinoma (HNSCC), such as oropharyngeal squamous cell carcinoma (OPSSC).

**BACKGROUND**

Head and neck squamous cell carcinomas (HNSCC) constitute 3-5% of all malignancies worldwide and there are approximately 600,000 newly diagnosed cases annually.[1,2] The majority of patients with HNSCC present with locally and/or regionally advanced disease at diagnosis. Despite the use of combined modality treatment, a significant proportion of patients develop unresectable recurrent or metastatic disease (R/M) HNSCC, for which treatment is palliative and consists of systemic therapy.[3-5] Even with the development of novel treatment regimens including immunotherapy, the median survival for patients with R/M HNSCC is typically less than one year.[6] There is an increasing incidence of human papillomavirus associated oropharyngeal squamous cell carcinoma (HPV+ OPSCC) and this represents one of only four cancers increasing in incidence in the United States.[7]

A number of prognostic clinical factors have been identified for patients with recurrent/metastatic (R/M) HNSCC including HPV status[8]. However, the only validated predictive biomarker is PD-L1, allowing for identification of patients who will have survival benefit with pembrolizumab versus chemotherapy as a first line treatment. Despite this, treatment response rates with currently available therapeutics remain low and no biomarker has been validated for dynamic response assessment or prediction of treatment benefit including PD-L1[9-11]. Given this, efficacy of therapy can only able to be assessed after 9-12 weeks of treatment since radiographic responses are often delayed.[6,9,11,12] As a result of the limited response rate, many patients experience significant progression leading to airway compromise and impaired functional status, rendering them unsuitable for further cancer directed therapy. A method is needed to rapidly identify those not benefiting from therapy, as well as to avoid progressive disease on a futile therapy and enable an earlier switch to a potentially efficacious treatment. A clear need exists for treatment individualization in R/M HNSCC, which necessitates identification of predictive biomarkers that can inform about response to therapy in real-time. Additionally, such an assay, may have utility for the detection of early recurrence for previously untreated patients, or for detection of response to therapy in early treatment settings.

The invention is set out in the appended claims, which differs from the prior art related to the detection of HVP16, including WO 2020/055834 A2 and Hanna et al., "Plasma HPV cell-free DNA monitoring in advanced HPV-associated oropharyngeal cancer", Annals of Oncology 29, pages 1980-1986, 2018, in the use of a different primer set and provides for HPV16 DNA detection with 100% specificity compared to HPV18 DNA and HPV negative samples.

**SUMMARY**

In some aspects, provided herein are methods of detecting HPV16 circulating tumor DNA (ctDNA) as defined in claim 1 and its dependent claims. Said methods for detecting HPV16 ctDNA comprise contacting a sample comprising cell free DNA with a set of oligonucleotides comprising a forward primer having at least 90% sequence identity to SEQ ID NO: 1, a reverse primer comprising SEQ ID NO: 2; and a probe comprising a detectable label. The forward primer and the reverse primer anneal to a target HPV16 sequence. The methods further comprise amplifying the target HPV16 sequence, if present in the sample, and detecting a signal from the detectable label. Detection of the signal indicates the presence of the target HPV16 sequence in the sample. In some embodiments, the probe comprises a fluorescent label.

In some aspects, the methods further comprise amplifying the target HPV16 sequence, if present in the sample, comprising the sequence of SEQ ID NO: 4 or a portion thereof comprising at least 15 continuous bases of SEQ ID NO:4, as defined in claim 3.

For any of the methods described herein, amplifying the target HPV16 sequence may be performed by quantitative PCR (qPCR). For example, the qPCR may be digital PCR. In some embodiments, the PCR is droplet digital PCR.

In some aspects, provided herein are methods of quantifying HPV16 circulating tumor DNA (ctDNA) as defined in claim 5 and its dependent claims. Said methods of quantifying HPV16 ctDNA comprise contacting a sample comprising cell free DNA with a set of oligonucleotides comprising a forward primer having at least 90% sequence identity to SEQ ID NO: 1, a reverse primer comprising SEQ ID NO: 2; and a probe comprising a detectable label. The forward primer and the reverse primer anneal to a target HPV16 sequence. The methods further comprise amplifying the target HPV16 sequence, if present in the sample, by droplet digital PCR. In some embodiments, the methods further comprise detecting a signal from the detectable label, wherein detection of the signal indicates the presence of the target HPV16 sequence. The methods further comprise quantifying the amount of the target HPV16 sequence present in the sample.

For any of the methods described herein, the probe may be an oligonucleotide having at least 90% sequence identity to SEQ ID NO: 3. The cell free DNA may be isolated form any suitable sample. In some embodiments, the cell free DNA is isolated from a plasma sample. In some embodiments, the cell free DNA is isolated from a saliva sample.

For any of the methods described herein, the forward primer may comprise the nucleotide sequence of SEQ ID NO: 1, the reverse primer comprises the nucleotide sequence of SEQ ID NO: 2, and the probe may comprise the nucleotide sequence of SEQ ID NO: 3.

In some aspects, provided herein is a set of oligonucleotides for amplifying and detecting a target HPV16 sequence. As defined in claim 8, the set of oligonucleotides comprises a forward primer having at least 90% sequence identity to SEQ ID NO: 1; a reverse primer comprising SEQ ID NO: 2; and a probe having at least 90% sequence identity to SEQ ID NO: 3.

In some embodiments, the forward primer comprises the nucleotide sequence of SEQ ID NO: 1. The reverse primer comprises the nucleotide sequence of SEQ ID NO: 2. In some embodiments, the probe comprises the nucleotide sequence of SEQ ID NO: 3. In some embodiments, the probe comprises a detectable label. In some embodiments, the detectable label is a fluorescent label.

In some aspects, provided herein are methods of predicting response to treatment in a subject with HPV positive head and neck squamous cell carcinoma (HPV+ HNSCC). As defined in claim 10, the method comprises measuring a baseline level HPV16 ctDNA in the subject, measuring a follow on level of HPV16 ctDNA following one or more treatment sessions in the subject, and predicting response to treatment in the subject. Said predicting response comprises predicting a positive response to treatment in the subject when a change from the baseline level to the follow on level is below a threshold value; or said predicting response comprises predicting a negative response to treatment in the subject when a change from the baseline level to the follow on level is above a threshold value.

In some embodiments, the baseline level is measured prior to any treatment in the subject. In some embodiments, the baseline level is measured following a first treatment cycle and the follow on level is measured following a second treatment cycle.

In some embodiments, measuring the baseline level and/or measuring the follow on level comprise the steps as outlined in items (i) to (iii) of claim 10. Said measuring the baseline level and measuring the follow on level comprises (i) contacting a sample comprising cell free DNA from the subject with a set of oligonucleotides comprising a forward primer having at least 90% sequence identity to SEQ ID NO: 1, a reverse primer comprising SEQ ID NO: 2; and a probe comprising a detectable label, wherein the forward primer and the reverse primer anneal to a target HPV16 sequence; (ii) amplifying the target HPV16 sequence, if present in the sample, by droplet digital PCR, and (iii) detecting a signal from the detectable label, wherein detection of the signal indicates the presence of the target HPV16 sequence; and quantifying the amount of the target HPV16 present in the sample.

For any of the methods of predicting response described herein, the probe may comprise a fluorescent label. The probe may be an oligonucleotide having at least 90% sequence identity to SEQ ID NO: 3. The cell free DNA may be isolated from any suitable sample, including a plasma sample or a saliva sample obtained from the subject. In some embodiments, the forward primer comprises the nucleotide sequence of SEQ ID NO: 1, the reverse primer comprises the nucleotide sequence of SEQ ID NO: 2, and the probe comprises the nucleotide sequence of SEQ ID NO: 3.

In some embodiments, the threshold value is 50%. In some embodiments, the threshold value is 60%.

A positive response to treatment may indicate a complete response to treatment, a partial response to treatment, or a stable disease state in the subject. A negative response to treatment may indicate disease progression in the subject. In some embodiments, the treatment is immunotherapy. In some embodiments, the HNSCC is oropharyngeal squamous cell carcinoma (OPSCC).

In some aspects, which are not covered by the claims but described for the sake of completeness herein, the present invention relates to methods of treating a subject with HPV positive head and neck squamous cell carcinoma (HPV+ HNSCC). The method may comprise measuring a baseline level HPV16 ctDNA in the subject, measuring a follow on level of HPV16 ctDNA following one or more treatment sessions in the subject, and modulating therapy based upon a change from baseline to the follow on level. Modulating therapy may comprise increasing the dose and/or frequency of therapy or providing an alternative or additional therapy to the subject when the change from baseline to the follow on level is above a threshold value. The baseline level may be measured prior to any treatment in the subject. The baseline level may be measured following a first treatment cycle and the follow on level is measured following a second treatment cycle.

Measuring the baseline level and measuring the follow on level may comprise obtaining a sample comprising cell free DNA from the subject, and contacting the sample with a set of oligonucleotides comprising a forward primer having at least 90% sequence identity to SEQ ID NO: 1, a reverse primer having at least 90% sequence identity to SEQ ID NO: 2; and a probe comprising a detectable label. The forward primer and the reverse primer may anneal to a target HPV16 sequence. The method may further comprise amplifying the target HPV16 sequence, if present in the sample, by droplet digital PCR, detecting a signal from the detectable label, wherein detection of the signal indicates the presence of the target HPV16 sequence, and quantifying the amount of the target HPV16 present in the sample.

Measuring the baseline level and measuring the follow on level may comprise obtaining a sample comprising cell free DNA from the subject, and contacting the sample with a set of oligonucleotides comprising a forward primer, a reverse primer, and a probe comprising a detectable label. The forward primer and the reverse primer anneal to a target HPV16 sequence. The method may further comprise amplifying the target HPV16 sequence, if present in the sample, by droplet digital PCR. The target HPV16 sequences may comprise the sequence of SEQ ID NO: 4 or a portion thereof comprising at least 15 contiguous bases of SEQ ID NO:4. The method may further comprise detecting a signal from the detectable label, wherein detection of the signal indicates the presence of the target HPV16 sequence. The method may further comprise quantifying the amount of the target HPV16 present in the sample.

For any of the methods of measuring the baseline and/or follow on level, the probe may comprise a fluorescent label. The probe may be an oligonucleotide having at least 90% sequence identity to SEQ ID NO: 3. The cell free DNA may be isolated from a suitable sample, including plasma or saliva obtained from the subject.

The forward primer may comprise the nucleotide sequence of SEQ ID NO: 1, the reverse primer may comprise the nucleotide sequence of SEQ ID NO: 2, and the probe may comprise the nucleotide sequence of SEQ ID NO: 3. The threshold value may be 50% or 60%. The treatment may be immunotherapy. The HNSCC may be oropharyngeal squamous cell carcinoma (OPSCC).

## DESCRIPTION OF THE DRAWINGS

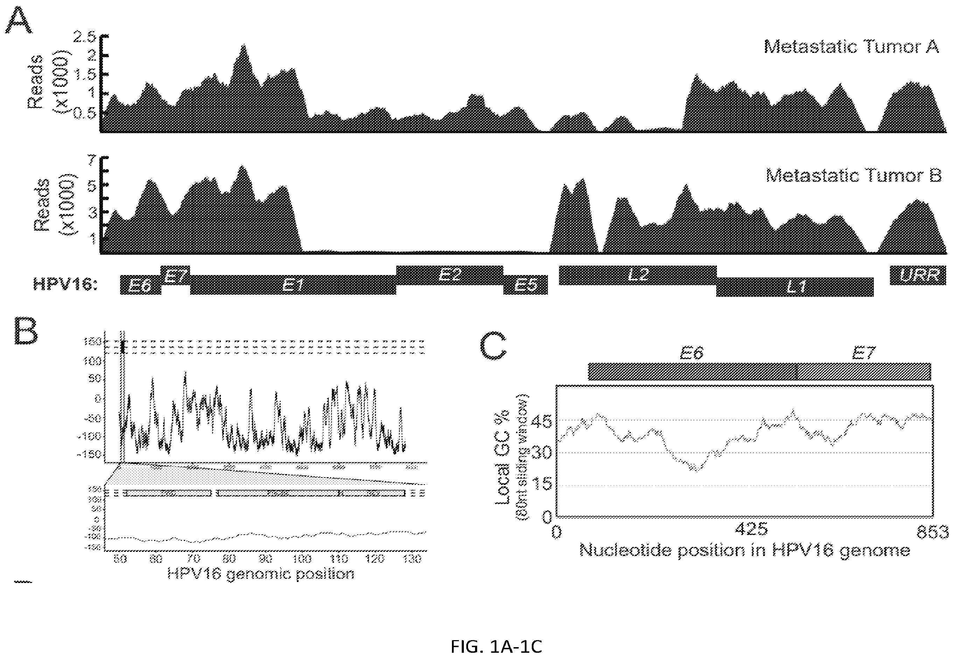

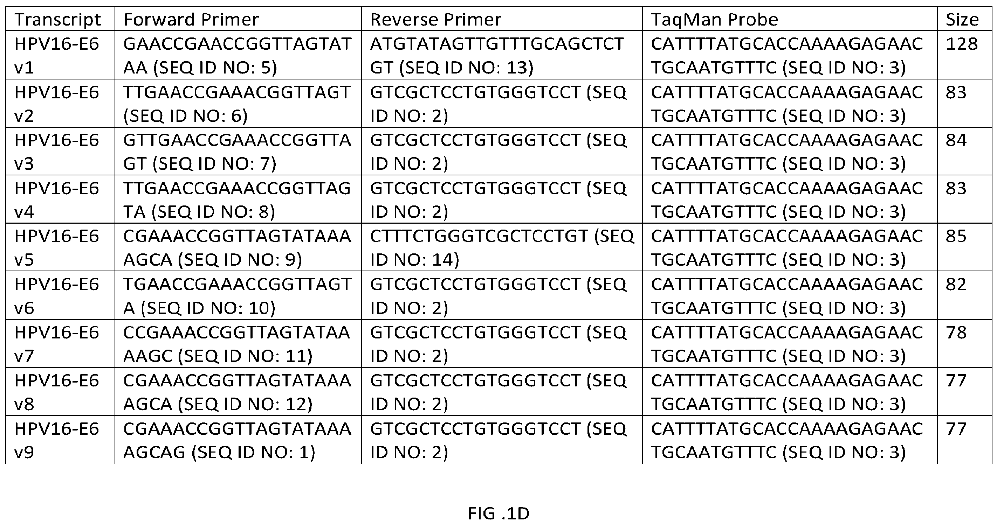

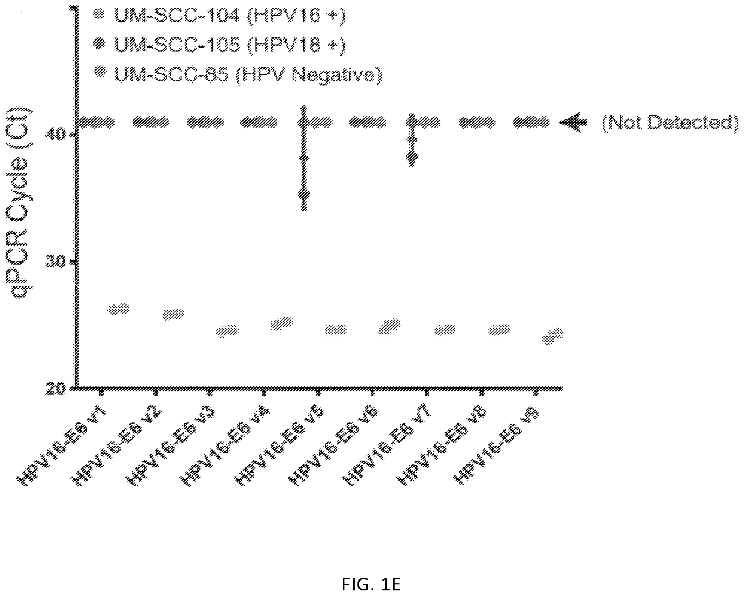

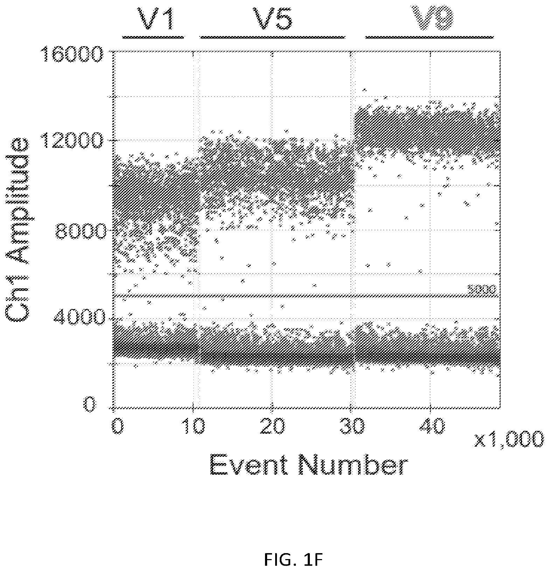

**Figure 1. HPV16 Plasma ctDNA Assay Development.** (A) Targeted capture NGS data for the two HPV16+ R/M HNSCC patients with sufficient biopsy material for sequencing are shown. High density HPV16 probes were used for capture, and the absolute read mapping to the reference HPV genome is shown. (B) A continuous black line is plotted, representing the maximum "global-local" alignment score (y-axis) calculated between the HPV18 genomic sequence and each 1 nt-offset 18-mer sequence extracted from the HPV16 genome. The scores are plotted corresponding to the start position of each window. The three horizontal dashed lines indicate the maximum pairwise alignment score for the 18-mer sequence with 0, 2 or 4 "N" mutations randomly introduced into the 18-mer reverse primer sequence. The lower plot zooms in on the region encompassing the expected PCR amplicon, and the locations of the forward, reverse and probe annealing sites are indicated. (C) Percent of GC-content was calculated within an 80nt sliding window. The genomic region of focus for assay design was plotted on the x-axis, such that the early region through nucleotide 853 (end of HPV16_E7) is shown. (D) Sequences of the nine different HPV16 primer-probe sets evaluated. (E) Results of amplification using each of the nine primer-probe sets on cell line genomic DNA (as indicated) was determined by quantitative, real-time PCR. UM-SCC-105, which is HPV18 positive was used as a specificity control, and UM-SCC-85, which is HPV negative, was used as a negative control. Ct value on the y-axis represents the cycle threshold value obtained in each case. (F) Three prioritized probes were evaluated on UM-SCC-104 cell line genomic DNA. Droplet generation, PCR, and droplet reading were performed and rain drop plots are shown.

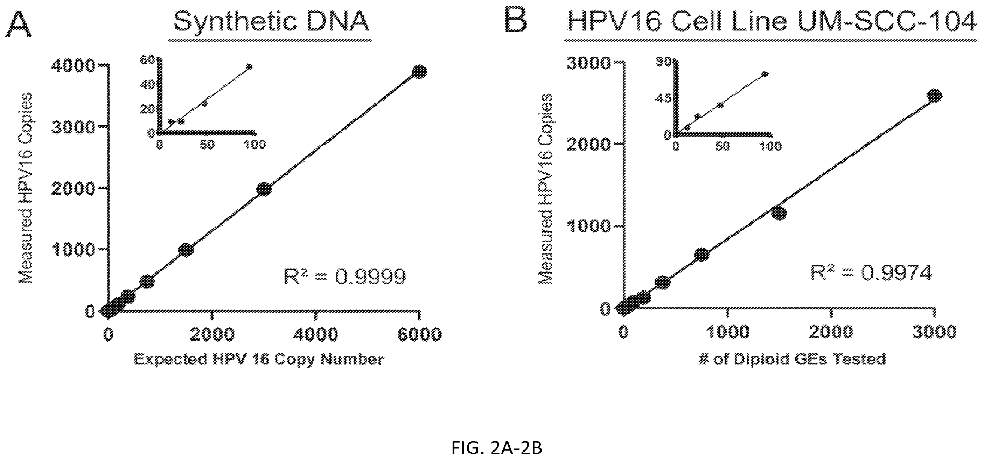

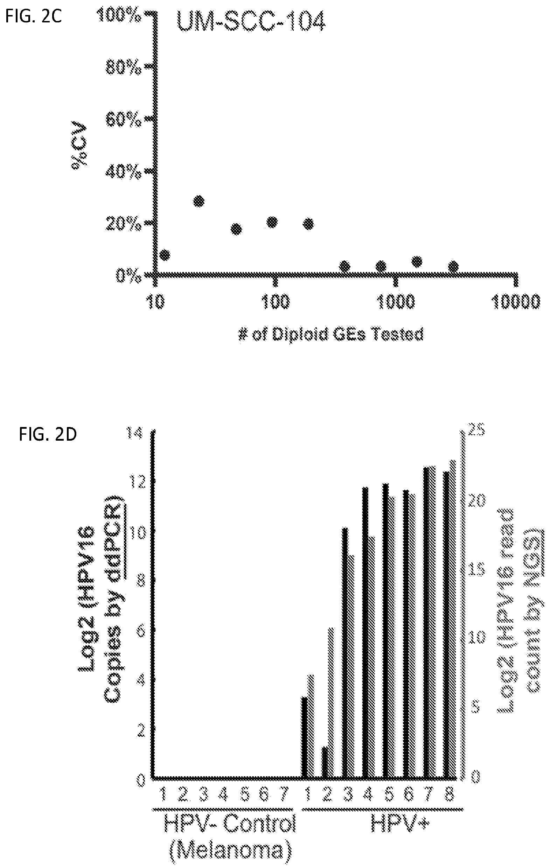

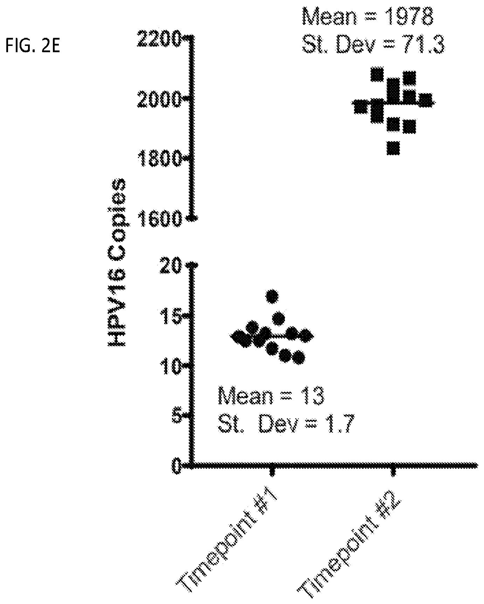

**Figure 2. Analytical Validation of HPV16 ctDNA droplet digital PCR assay.** (A) Plot shows ddPCR assay results from a 2-fold dilution series (cumulative of 3 replicates) to determine the limit of detection (LOD) and reportable range of *HPV16 E6* ctDNA. The expected copies of a 87bp synthetic DNA fragment containing the 77bp amplicon region of the *HPV16 E6* V9 assay, spiked into a human genomic DNA matrix (600,000 diploid GEs per data point) are shown on the *x-axis,* with the number of copies measured by the ddPCR analysis indicated on the *y-axis.* (B) The expected diploid GEs of *HPV16 E6* in UM-SCC-104 cell line DNA (*x-axis*) is plotted against the number of copies measured by the ddPCR a (*y-axis;* cumulative of 3 replicates). Based on the data shown, the LOD in both dilution series analyses (i.e., synthetic *HPV16 E6* DNA fragment in *(A)* and UM-SCC-104 cell line DNA in *(B)*) was calculated to be <5 copies per 20 uL reaction (see Materials and Methods). No positive droplets were observed when only *HindIII* digested human genomic DNA (200,000 diploid GEs per 20 ul reaction) was used as a non-HPV template (n = 15). (C) The plot shows % CV observed for *HPV16_E6* measured copy number at different dilutions plotted against the number of diploid GEs (converted to $\log_{10}$) of the UM-SCC-104 cell line DNA that was tested. (D) Tumor DNA extracted from FFPE-tumor specimens was analyzed with the HPV16 ddPCR assay. 7 melanomas were used as absolute HPV- samples and compared to 8 p16+ HNSCC tumors using the ddPCR assay (black bars), and compared to the HPV read counts from NGS (blue bars). (E) Multiple aliquots of plasma from one HPV16+ HNSCC patients obtained at two separate time points, with low and high ctDNA levels, were used for analysis of variance in the sample processing and analysis protocol.

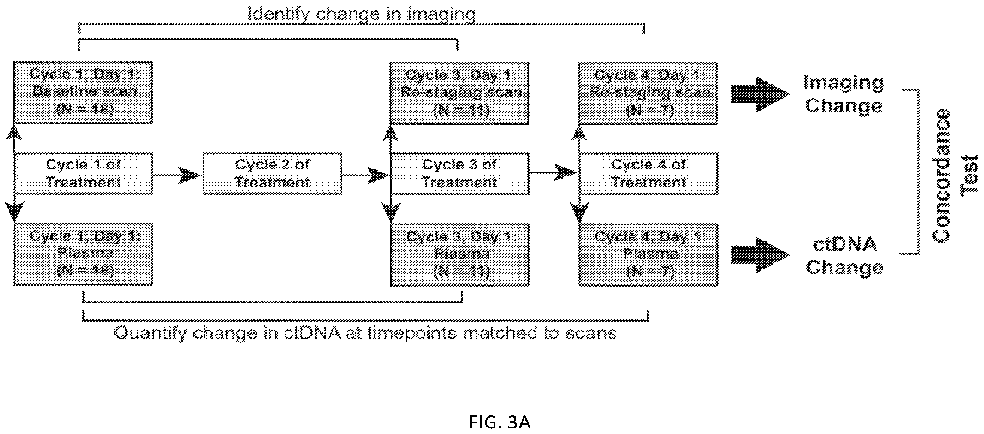

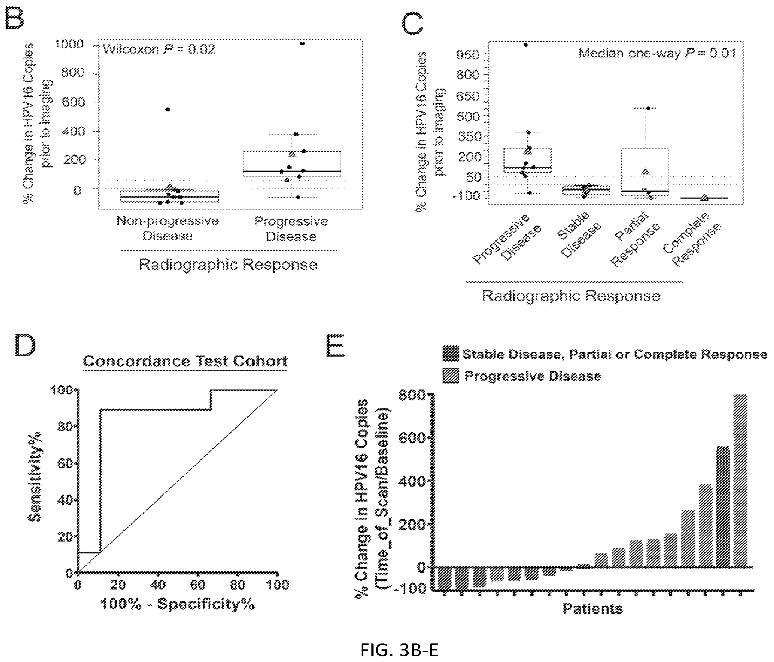

**Figure 3. Time point matched Changes in HPV16 ctDNA Copies are Highly Concordant with Changes in Radiographic Imaging in Recurrent and Metastatic HNSCC patients.** (A) Schematic timeline representation of the cohort to evaluate the correlation of change in HPV16 ctDNA and change in imaging. The change in radiographic response between each patient's baseline and re-staging CT scans (top bars) were compared to change in HPV16 ctDNA copies between a sample collected synchronously with imaging and a baseline sample (bottom bar). For this cohort, plasma was obtained on imaging matched time points for 18 total treatment series. (B) Box-plot analysis. A Wilcoxon test was performed to assess the difference between percent change in HPV16 ctDNA in patients with progressive disease (PD) and those deriving benefit (non-PD) on restaging imaging. The dotted line indicates a 60% change as identified in ROC curve analysis. (C) Box-plot analysis. Median one-way analysis tests were performed to evaluate for differences in percent change of HPV ctDNA between patients with progressive disease (PD), stable disease (SD), partial response (PR), and complete response (CR) on restaging imaging. Median one-way analysis demonstrated statistically significant changes across response categories (p=0.01). The dotted line indicates a 60% change as identified in ROC curve analysis. The two purple circles highlight patients whom were found to have pseudoprogression. (D) ROC curve analysis was performed and identified a $\geq$ 60% increase in HPV16 ctDNA being associated with the optimal sensitivity and specificity for predication of progression on radiographic imaging. (E) A waterfall plot demonstrates each patient's percent change in HPV16 ctDNA level relative to baseline at the time of re-imaging. Color coding indicates radiographic response as indicated.

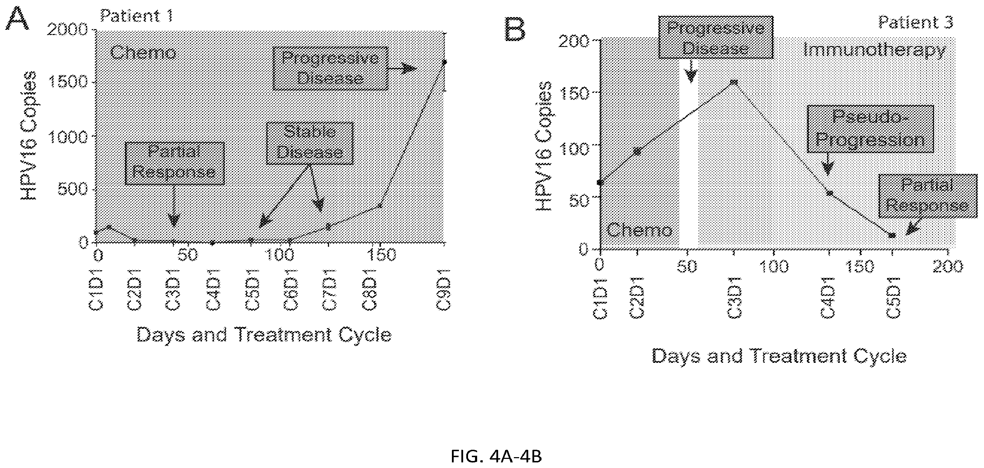

**Figure 4A-C. Patient HPV16 ctDNA Levels and Treatment Histories.** Plasma HPV16 ctDNA levels (copies per $1 \mu L$) measured over time in patients with p16+ R/M HPV+ OPSCC (A-C). On the X-axis is days since study enrollment as well as cycle and day (CxDx) of treatment cycle. Colored boxes represent treatment courses (chemo=chemotherapy, immune=immunotherapy). Radiographic response are noted in each graph. **(A)** highlights HPV16 ctDNA identifying progression more than 100 days prior to radiographic imaging. **(B) and (C)** highlight patients with radiographic pseudoprogression while treated with immunotherapy. Select serial CT images are shown for patient 10 at baseline, identification of pseudoprogression, and confirmatory imaging demonstrating partial response. A left pleural based soft tissue metastasis (top row) was initially noted to increase in size abutting the mediastinum, prior to decreasing in size dramatically. Similarly, a small sub-centimeter pleural based nodule (bottom row) initially grew to 1.1 cm prior to resolving completely on confirmatory imaging.

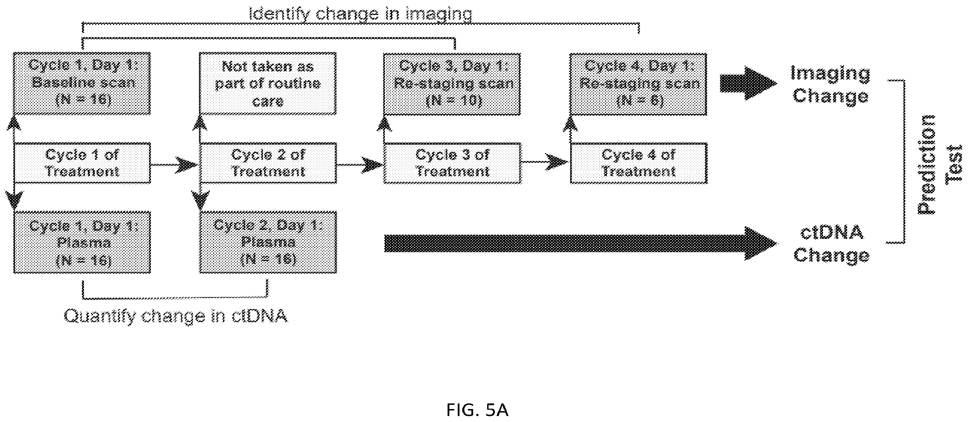

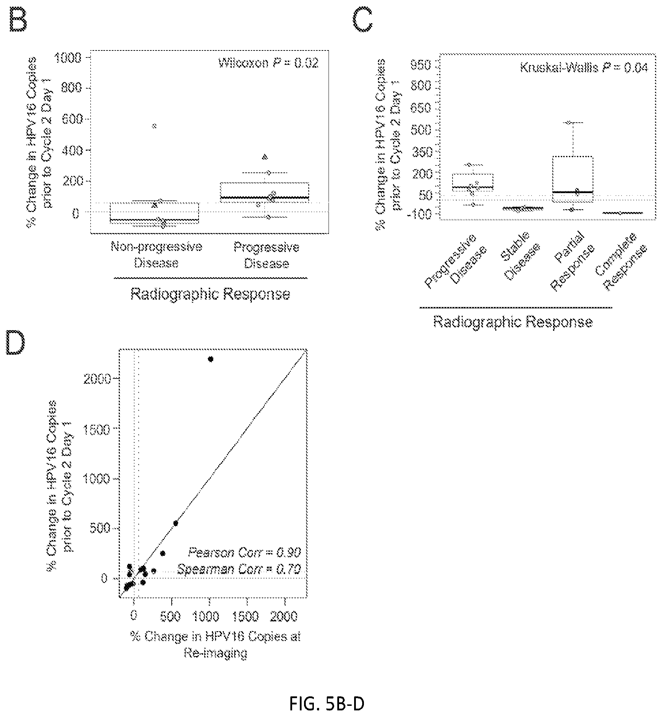

**Figure 5. The Absolute Change in HPV16 ctDNA Copies After One Cycle of Treatment Predicts Radiographic Response in Recurrent and Metastatic HNSCC patients.** (A) Schematic timeline representation of the design of the cohort to evaluate the predictive value of HPV16 ctDNA after one cycle of treatment. Change in radiographic response between each patient's baseline and re-staging CT scans (top bars) were compared to change in HPV16 ctDNA copies between the post-cycle 1 of treatment time point and baseline (bottom bar). For this cohort, plasma was obtained after the first cycle of treatment for 16 of the 18 potential treatment series as two patients missed blood collection; therefore, the total N analyzed is 16. (B) Box-plot analysis. Wilcoxon test was performed to assess the difference between percent change in HPV16 ctDNA after one cycle of treatment in patients with progressive disease (PD) and those deriving benefit (non-PD). The dotted line indicates a 60% change as identified in previous ROC curve analysis. (C) Box-plot analysis. The Kruskal-Wallis analysis test were performed to evaluate for differences in percent change of HPV ctDNA after one cycle of treatment between patients with progressive disease (PD), stable disease (SD), partial response (PR), and complete response (CR) on restaging imaging. Kruskal-Wallis test demonstrated statistically significant changes across response categories (p=0.04). The dotted line indicates a 60% change. The purple circles highlight patients with pseudoprogression. (D) Scatter plot of percent change in HPV16 ctDNA observed in the draw after one cycle of treatment and the percent change in the blood drawn synchronous with restaging imaging. Spearman and Pearson's rho is reported to assess the magnitude of correlation.

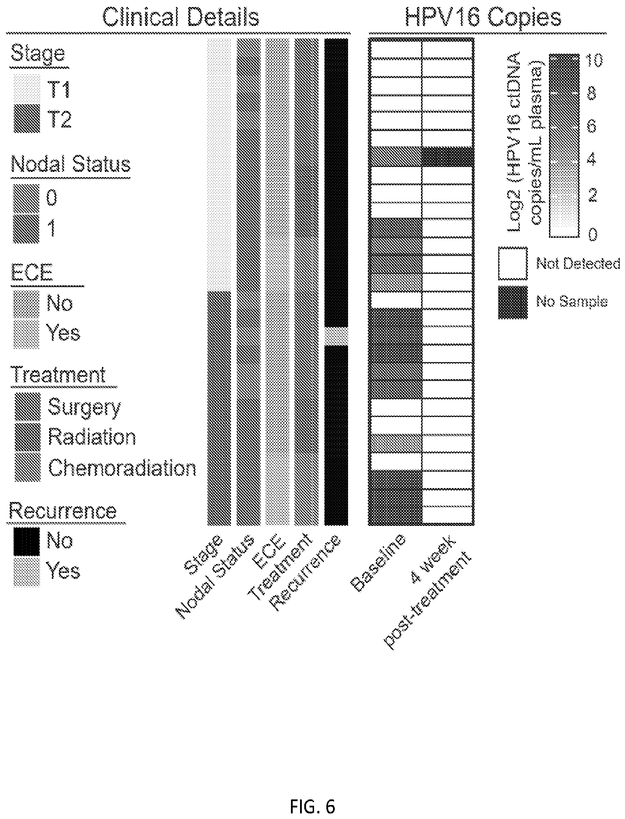

**Figure 6. HPV16 ctDNA is undetectable in low stage disease as soon as 4 weeks after treatment.** Plasma samples from patients were analyzed before and after treatment. Heatmap shows the absolute quantification of HPV16 ctDNA copies detected in each sample. Clinical variables associated with each patient, including T and N stage (AJCC 8), extracapsular extension (ECE) and treatment strategy are shown. Note that one patient with detectable baseline ctDNA missed the 1 month draw.

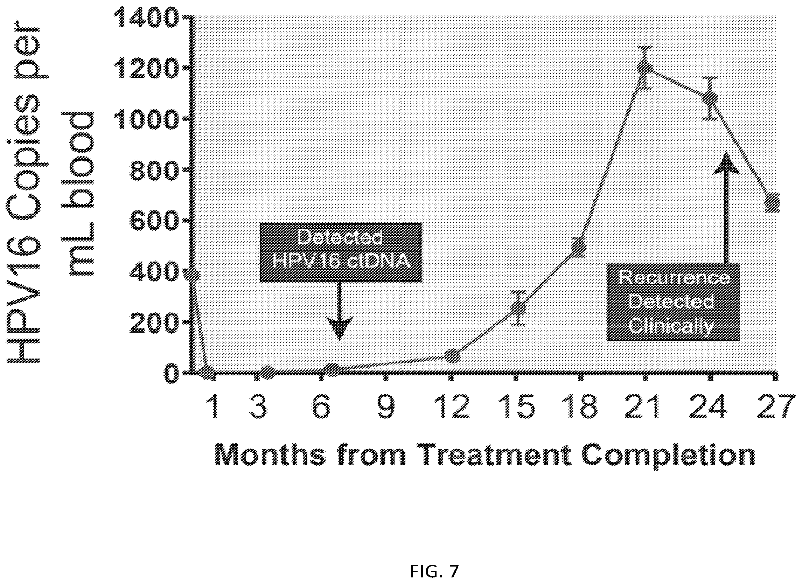

**Figure 7. Longitudinal ctDNA quantification in recurrent patient.** Ten plasma samples drawn from pre-treatment to 2.5 years post-treatment were analyzed for HPV16 ctDNA, and all samples except the 4 week and 3 month time points had detectable HPV16 ctDNA copies.

## DEFINITIONS

Although any methods and materials similar or equivalent to those described herein can be used in the practice or testing of embodiments described herein, some preferred methods, compositions, devices, and materials are described herein. However, before the present materials and methods are described, it is to be understood that this invention is not limited to the particular molecules, compositions, methodologies, or protocols herein described, as these may vary in accordance with routine experimentation and optimization. It is also to be understood that the terminology used in the description is for the purpose of describing the particular versions or embodiments only.

Unless otherwise defined, all technical and scientific terms used herein have the same meaning as commonly understood by one of ordinary skill in the art to which this invention belongs. However, in case of conflict, the present specification, including definitions, will control. Accordingly, in the context of the embodiments described herein, the following definitions apply.

As used herein and in the appended claims, the singular forms "a", "an" and "the" include plural reference unless the context clearly dictates otherwise.

As used herein, the term "comprise" and linguistic variations thereof denote the presence of recited feature(s), element(s), method step(s), etc. without the exclusion of the presence of additional feature(s), element(s), method step(s), etc. Conversely, the term "consisting of" and linguistic variations thereof, denotes the presence of recited feature(s), element(s), method step(s), etc. and excludes any unrecited feature(s), element(s), method step(s), etc., except for ordinarily-associated impurities. The phrase "consisting essentially of" denotes the recited feature(s), element(s), method step(s), etc. and any additional feature(s), element(s), method step(s), etc. that do not materially affect the basic nature of the composition, system, or method. Many embodiments herein are described using open "comprising" language. Such embodiments encompass multiple closed "consisting of" and/or "consisting essentially of" embodiments, which may alternatively be claimed or described using such language.

An "amplicon" refers to a nucleic acid fragment formed as a product of natural or artificial amplification events or techniques. For example, an amplicon can be produced by PCR.

As used herein, the terms "subject" and "patient" are used interchangeably herein and refer to both human and nonhuman animals. The term "nonhuman animals" includes all vertebrates, e.g., mammals and non-mammals, such as nonhuman primates, sheep, dogs, cats, horses, cows, chickens, amphibians, reptiles, and the like. In some embodiments, the subject is a human. In particular embodiments, the subject may be male. In other embodiments, the subject may be female. In some embodiments, the subject is suffering from cancer.

As used herein, "treat", "treating", "treatment", and variations thereof refer to the clinical intervention made in response to a disease, disorder or physiological condition manifested by a patient or to which a patient may be susceptible. The aim of treatment includes the alleviation or prevention of symptoms, slowing or stopping the progression or worsening of a disease, disorder, or condition and/or the remission of the disease, disorder, or condition. For example, treating a cancer refers to the management and care of the subject for combating and reducing one or more symptoms of the cancer. For example, treating cancer may reduce tumor burden (e.g. reduce the size of one or more tumors in the subject afflicted with cancer and/or reduce the overall number of tumors in the subject afflicted with cancer). Treating a cancer may reduce or completely eliminate the cancer (e.g. completely eliminate the tumor) in the subject.

## DETAILED DESCRIPTION

Solid tumors are known to shed circulating tumor DNA (ctDNA) which might be detectable bodily fluids including saliva, urine, and plasma.[13-15] Plasma ctDNA analyses allow for non-invasive longitudinal monitoring of tumor specific genomic alterations. To date a comprehensive prospective analysis of ctDNA assay characteristics and predictive efficacy has not been performed in head and neck cancer.

Described herein is the development of an HPV16 ctDNA test that offers a precise assay for detection of HPV+ OPSCC. Moreover, the assay described herein finds use to predict progressive disease prior to radiographic imaging in patients undergoing treatment. A highly sensitive and specific droplet digital PCR (ddPCR) assay for absolute quantification of HPV ctDNA from plasma specimens is described herein.

For any of the methods described herein, any suitable sample type may be used. The sample may be obtained from the subject and subsequently used for any of the methods described herein. In some embodiments, the sample is obtained from the subject and circulating tumor DNA is isolated from the sample for use in the methods described herein. Suitable samples include fluids (e.g. urine, blood, blood products, sputum, saliva, etc.), solids, tissues, and gases. In some embodiments, the sample is blood (e.g. whole blood) or a blood product such as plasma, serum, and the like. In some embodiments, the sample is a plasma sample. In some embodiments, the sample is a sample obtained from the mouth of the subject. For example, the sample may be a saliva sample. The term "saliva" or "saliva sample" is meant to include any sample containing saliva from the subject, including spit, an oral swab or sponge sample, a mouthwash rinse sample, etc.

**Methods of Detecting and/or Quantifying HPV ctDNA**

In some aspects, provided herein are methods of detecting HPV circulating tumor DNA (ctDNA) as defined in the claims. In some aspects, provided herein are methods for detecting HPV subtype 16 (HPV16) ctDNA as defined in the claims. The methods comprise contacting a sample comprising cell free DNA with a set of oligonucleotides as defined in the claims. As defined in the claims, the set of oligonucleotides comprises a forward primer, a reverse primer, and a probe.

The set of oligonucleotides may generate an amplicon comprising the sequence of: CGAAACCGGTTAGTAT AAAAGCAGACATTTTATGCACCAAAAGAG AACTGCAATGTTTCAGGACCCACAGGAGCGAC (SEQ ID NO: 4), or a portion thereof having at least 15 contiguous bases of SEQ ID NO:4 (e.g., at least 15, 18, 20, 25, 30, etc.). The set of oligonucleotides may comprise a forward primer and a reverse primer that generate an amplicon of SEQ ID NO: 4 or a portion thereof, and the probe comprises an oligonucleotide capable of hybridizing to the amplicon.

The forward primer has at least 90% sequence identity to CGAAACCGGTTAGTATAAAAGCAG (SEQ ID NO: 1). For example, the forward primer may have at least 90%, at least 91%, at least 92%, at least 93%, at least 94%, at least 95%, at least 96%, at least 97%, at least 98%, at least 99%, or 100% sequence identity to SEQ ID NO: 1. The reverse primer comprises GTCGCTCCTGTGGGTCCT (SEQ ID NO: 2).

The probe comprises a detectable label. Any suitable detectable label may be used. In some embodiments, the detectable label is a fluorescent label. Suitable fluorescent labels include, for example, FAM (5- or 6-carboxyfluorescein), VIC, NED, Fluorescein, FITC, IRD-700/800, CY3, CY5, CY3.5, CY5.5, HEX, TET, TAMRA, JOE, ROX, BODIPY TMR, Oregon Green, Rhodamine Green, Rhodamine Red, Texas Red, Yakima Yellow, Alexa Fluor PET, Biosearch Blue™, Marina Blue®, Bothell Blue®, Alexa Fluor®, 350 FAM™, SYBR® Green 1, Fluorescein, EvaGreen™, Alexa Fluor® 488 JOE™, VIC™ HEX™ TET™, CAL Fluor® Gold 540, Yakima Yellow®, ROX™, CAL Fluor® Red 610, Cy3.5™, Texas Red®, Alexa Fluor® 0.568 Cy5™, Quasar™ 670, LightCycler Red640®, Alexa Fluor 633 Quasar™ 705, LightCycler Red705®, Alexa Fluor® 680, SYTO® 9, LC Green®, LC Green® Plus+, and EvaGreen™.

In some embodiments, the probe comprises an oligonucleotide having at least 90% sequence identity to CA TTTTATGCACCAAAAGAGAACTGCAATGTTTC (SEQ ID NO: 3). For example, the probe may comprise an oligonucleotide having at least 90%, at least 91%, at least 92%, at least 93%, at least 94%, at least 95%, at least 96%, at least 97%, at least 98%, at least 99%, or 100% sequence identity to SEQ ID NO: 3.

The forward primer and the reverse primer anneal to a target HPV16 sequence (e.g. a target region within the HPV16 genome). HPVs, including HPV16, contain a 7.9-kb circular double-stranded DNA genome that consists of four parts: an early region (E1, 2, 4-7 genes), a late region (L1, 2 genes), a long control region (LCR), and a small, highly variable, non-coding region (NCR) between E5 and L2. The early region and late region of HPV16 are shown in FIG. 1A. In some embodiments, the forward primer and the reverse primer anneal to a target sequence within the E6 gene. In some embodiments, the forward primer and the reverse primer anneal to and amplifies, if present, a 77 bp region (e.g. an amplicon) within the E6 gene of HPV16. In some embodiments, the forward primer and the reverse primer anneal to and amplify, if present, the amplicon of SEQ ID NO: 4 or a portion thereof.

In some embodiments, the method comprises contacting a sample comprising cell free DNA with a set of oligonucleotides comprising a forward primer having at least 90% sequence identity to SEQ ID NO: 1, a reverse primer comprising SEQ ID NO: 2; and a probe comprising an oligonucleotide having at least 90% sequence identity to SEQ ID NO: 3 and a detectable label. In some embodiments, the forward primer comprises the nucleotide sequence of SEQ ID NO: 1, the reverse primer comprises the nucleotide sequence of SEQ ID NO: 2, and the probe comprises the nucleotide sequence of SEQ ID NO: 3.

The methods further comprise amplifying the target HPV16 sequence, if present in the sample. The methods further comprise detecting a signal from the detectable label, which is indicative of the presence of the target HPV16, if present in the sample. For example, amplification may be performed by polymerase chain reaction (PCR). In some embodiments, the PCR is a quantitative PCR (qPCR). For example, amplification of the target sequence may be performed using a digital PCR technique, such as a dPCR technique selected from droplet digital PCR (ddPCR), BEAMing (beads, emulsion, amplification, and magnetic), and microfluidic chips.

As used herein, "digital PCR" refers to an assay that provides an end-point measurement that provides the ability to quantify nucleic acids without the use of standard curves, as is used in real-time PCR. In a typical digital PCR experiment, the sample is randomly distributed into discrete partitions, such that some contain no nucleic acid template and others contain one or more template copies. The partitions are amplified to the terminal plateau phase of PCR (or end-point) and then read to determine the fraction of positive partitions. If the partitions are of uniform volume, the number of target DNA molecules present may be calculated from the fraction of positive end-point reactions using Poisson statistics, according to the following equation:

$$\lambda = -1n(1-p) \qquad (1)$$

wherein $\lambda$ is the average number of target DNA molecules per replicate reaction and p is the fraction of positive end-point reactions. From k, together with the volume of each replicate PCR and the total number of replicates analyzed, an estimate of the absolute target DNA concentration is calculated. Digital PCR includes a variety of formats, including droplet digital PCR, BEAMing (beads, emulsion, amplification, and magnetic), and microfluidic chips.

In some embodiments, the dPCR is droplet digital PCR. "Droplet digital PCR" (ddPCR) refers to a digital PCR assay that measures absolute quantities by counting nucleic acid molecules encapsulated in discrete, volumetrically defined, water-in-oil droplet partitions that support PCR amplification (Hinson et al., 2011, Anal. Chem. 83:8604-8610; Pinheiro et al., 2012, Anal. Chem. 84:1003-1011). A single ddPCR reaction may be comprised of at least 20,000 partitioned droplets per well. A "droplet" or "water-in-oil droplet" refers to an individual partition of the droplet digital PCR assay. A droplet supports PCR amplification of template molecule(s) using homogenous assay chemistries and workflows similar to those widely used for real-time PCR applications (Hinson et al., 2011, Anal. Chem. 83:8604-8610; Pinheiro et al., 2012, Anal. Chem. 84:1003-1011).

Droplet digital PCR may be performed using any platform that performs a digital PCR assay that measures absolute quantities by counting nucleic acid molecules encapsulated in discrete, volumetrically defined, water-in-oil droplet partitions that support PCR amplification. The strategy for droplet digital PCR may be summarized as follows: a sample is diluted and partitioned into thousands to millions of separate reaction chambers (water-in-oil droplets) so that each contains one or no copies of the nucleic acid molecule of interest. The number of "positive" droplets detected, which contain the target amplicon (i.e., nucleic acid molecule of interest), versus the number of "negative" droplets, which do not contain the target amplicon (i.e., nucleic acid molecule of interest), may be used to determine the number of copies of the nucleic acid molecule of interest that were in the original sample. Examples of droplet digital PCR systems include the QX100™ Droplet Digital PCR System by Bio-Rad, which partitions samples containing nucleic acid template into 20,000 nanoliter-sized droplets; the QX200™ Droplet Digital PCR System by Bio-Rad; and the RainDrop™ digital PCR system by RainDance, which partitions samples containing nucleic acid template into 1,000,000 to 10,000,000 picoliter-sized droplets.

In some embodiments, the method further comprises quantifying HPV16 ctDNA in the sample.

In some aspects, provided herein is a method for quantifying HPV16 ct DNA in a sample. The method may be substantially the same as the above-described methods for detecting HPV16 ctDNA in the sample, and further comprise quantifying the HPV16 ctDNA, if present in the sample. In some embodiments, the method comprises contacting a sample comprising cell free DNA with a set of oligonucleotides comprising a forward primer having at least 90% sequence identity to SEQ ID NO: 1, a reverse primer comprising SEQ ID NO: 2; and a probe comprising a detectable label. In some embodiments, the method further comprises amplifying the target HPV16 sequence, if present in the sample, by droplet digital PCR.

In some embodiments, the target HPV16 sequence comprises SEQ ID NO: 4 or a portion thereof having at least 15 contiguous bases of SEQ ID NO:4. For example, in some embodiments the method comprises contacting a sample comprising cell free DNA with a set of oligonucleotides capable of generating an amplicon comprising SEQ ID NO: 4 or a portion thereof. For example, in some embodiments the set of oligonucleotides comprise a forward primer and a reverse primer that generates an amplicon comprising SEQ ID NO: 4 or a portion thereof, and a probe that hybridizes to the amplicon.

The method further comprises detecting a signal from the detectable label, wherein detection of the signal indicates the presence of the target HPV16 sequence, and quantifying the amount of the target HPV16 present in the sample. Quantification may be performed, for example, by determining the number of target DNA molecules present. The number of target DNA molecules present may be calculated from the fraction of positive end-point reactions using Poisson statistics, as described above.

The methods of detecting and/or quantifying HPV16 ctDNA may be used in a variety of diagnostic or predictive methods. In some embodiments, the methods of detecting or the methods of quantifying HPV16 ctDNA may be used in methods of determining whether a subject has an HPV positive cancer. For example, the methods may be used to determine whether a subject has an HPV positive cervical cancer, head and neck cancer (e.g. oropharyngeal cancer), anal cancer, penile cancer, vaginal cancer, or vulvar cancer. For example, the methods may be used to determine whether a subject has an HPV positive head and neck cancer, such as HPV positive oropharyngeal cancer.

The methods for detecting or the methods for quantifying HPV16 ctDNA may be used in methods of predicting patient response to therapy in a cancer. Such methods are described in further detail below.

## A. Methods of Predicting Response

In some aspects, provided herein are methods of predicting response to treatment in a subject. In some embodiments, provided herein are methods for predicting response to treatment in a subject suffering from cancer. The treatment may be any suitable treatment/therapy for cancer. Suitable therapies include, for example, surgery, chemotherapy, radiation therapy, targeted therapy, immunotherapy, stem cell transplant therapy, bone marrow transplant therapy, or hormone therapy. The treatment may be immunotherapy. As used herein, the term "immunotherapy" refers to any type of cancer treatment that helps the immune system fight cancer. Said "immunotherapy" may refer to treatment with an immune checkpoint inhibitor, T-cell transfer therapy, monoclonal antibodies, treatment vaccines, and/or immune modulators. The cancer may be any cancer type. The cancer may be an HPV positive cancer. For example, the cancer may be an HPV positive cancer selected from cervical cancer, head and neck cancer (e.g. oropharyngeal cancer), anal cancer, penile cancer, vaginal cancer, or vulvar cancer. For example, in some embodiments provided herein is a method of predicting response immunotherapy in a subject suffering from HPV positive head and neck squamous cell carcinoma. For example, the HPV positive head and neck squamous cell carcinoma maybe HPV positive oropharyngeal squamous cell carcinoma.

In some embodiments, the method of predicting response comprises measuring a baseline level HPV16 ctDNA in the subject. The baseline level of HPV16 ctDNA may be measured prior to any treatment in the subject. Alternatively, the baseline level of HPV16 ctDNA may be measured following one or more treatment sessions in the subject. For example, the cancer therapy (e.g., immunotherapy) may be provided to the subject over the course of one or more cycles. Each cycle may comprise a single immunotherapy session (e.g. dose) or multiple sessions (e.g. multiple doses, over the course of multiple days). In some embodiments, the baseline level is measured after a therapy (e.g. an immunotherapy) cycle.

The method further comprises measuring a follow on level of HPV16 ctDNA following one or more treatment sessions in the subject. For example, a baseline level of HPV16 ctDNA may be measured prior to any therapy, and a follow on level may be measured following a therapy cycle. Alternatively, a baseline level of HPV16 ctDNA may be measured after a first therapy cycle, and a follow on level may be measured following a second therapy cycle.

The baseline level and/or follow on level of HPV16 ctDNA may be measured using a suitable method described herein. In some embodiments, measuring the baseline level and measuring the follow on level comprises contacting a sample comprising cell free DNA from the subject with a set of oligonucleotides comprising a forward primer having at least 90% sequence identity to SEQ ID NO: 1, a reverse primer comprising SEQ ID NO: 2; and a probe comprising a detectable label. In some embodiments, the method comprises contacting a sample comprising cell free DNA with a set of oligonucleotides capable of generating an amplicon comprising SEQ ID NO: 4 or a portion thereof.

In some embodiments, the detectable label is a fluorescent label. Suitable fluorescent labels are described above. In some embodiments, the probe is an is an oligonucleotide having at least 90% sequence identity to SEQ ID NO: 3. In some embodiments, forward primer and the reverse primer anneal to a target HPV16 sequence. The method may further comprise amplifying the target HPV16 sequence, if present in the sample, by droplet digital PCR. In some embodiments, the method further comprises detecting a signal from the detectable label, wherein detection of the signal indicates the presence of the target HPV16 sequence. In some embodiments, the method further comprises quantifying the amount of the target HPV16 present in the sample.

The cell free DNA may be isolated from plasma obtained from the subject. For example, plasma may be obtained and cell free DNA may be isolated using any suitable method. In some embodiments, cell free DNA is isolated using a commercially available kit.

The method further involves predicting response to therapy in the subject based upon HPV16 ctDNA levels. In some embodiments, the method comprises predicting a positive response to treatment in the subject when a change from the baseline level to the follow on level is below a threshold value. In some embodiments, the method comprises predicting a negative response to treatment in the subject when a change from the baseline level to the follow on level is above a threshold value. In some embodiments, the threshold value is 50%. In some embodiments, the threshold value is 60%. For example, the method may comprise predicting a positive response to treatment when there is less than a 60% increase in HPV16 ctDNA levels from the baseline level to the follow on level. Alternatively, the method may comprise predicting a negative response to treatment when there a 60% or higher increase in HPV16 ctDNA levels from the baseline level to the follow on level. A positive response to treatment may indicate a complete response to treatment, a partial response to treatment, or a stable disease state in the subject. For example, a positive response may indicate no metastasis, no increase in tumor size, and/or no increase in total number of tumors observed in the subject. A negative response may indicate progression of the disease, such as increased total number of tumors and/or increased tumor size.

## B. Methods of Treating Cancer

In some aspects, which are not covered by the claims bur described for the sake of completeness herein, the present invention relates to methods of treating cancer in a subject. The subject may be undergoing or about to begin a suitable treatment/therapy for cancer. Suitable therapies include, for example, surgery, chemotherapy, radiation therapy, targeted therapy, immunotherapy, stem cell transplant therapy, bone marrow transplant therapy, or hormone therapy. The treatment may be immunotherapy. As used herein, the term "immunotherapy" refers to any type of cancer treatment that helps the immune system fight cancer. Said "immunotherapy" may refer to treatment with an immune checkpoint inhibitor, T-cell transfer therapy, monoclonal antibodies, treatment vaccines, and/or immune modulators. The cancer may be any cancer type. The cancer may beis an HPV positive cancer. For example, the cancer may be an HPV positive cancer selected from cervical cancer, head and neck cancer (e.g. oropharyngeal cancer), anal cancer, penile cancer, vaginal cancer, or vulvar cancer. For example, provided herein is a method of treating a subject suffering from HPV positive head and neck squamous cell carcinoma. For example, the HPV positive head and neck squamous cell carcinoma maybe HPV positive oropharyngeal squamous cell carcinoma.

The method of treating the subject may comprise measuring a baseline level HPV16 ctDNA in the subject. The baseline level of HPV16 ctDNA may be measured prior to any treatment in the subject. Alternatively, the baseline level of HPV16 ctDNA may be measured following one or more treatment sessions in the subject. For example, the cancer therapy (e.g., immunotherapy) may be provided to the subject over the course of one or more cycles. Each cycle may comprise a single immunotherapy session (e.g. dose) or multiple sessions (e.g. multiple doses, over the course of multiple days). The baseline level may be measured after a therapy (e.g. an immunotherapy) cycle. The method further comprises measuring a follow on level of HPV16 ctDNA following one or more treatment sessions in the subject. For example, a baseline level of HPV16 ctDNA may be measured prior to any therapy, and a follow on level may be measured following a therapy cycle. Alternatively, a baseline level of HPV16 ctDNA may be measured after a first therapy cycle, and a follow on level may be measured following a second therapy cycle.

The baseline level and/or follow on level of HPV16 ctDNA may be measured using any suitable method. The baseline level and/or follow on level may be measured using a method described herein. Measuring the baseline level and measuring the follow on level may comprise obtaining a sample comprising cell free DNA from the subject. The method may further comprise contacting the sample with a set of oligonucleotides comprising a forward primer having at least 90% sequence identity to SEQ ID NO: 1, a reverse primer comprising SEQ ID NO: 2; and a probe comprising a detectable label. The method may comprise contacting a sample comprising cell free DNA with a set of oligonucleotides capable of generating an amplicon comprising SEQ ID NO: 4 or a portion thereof.

The probe may comprise a detectable label. The detectable label may be a fluorescent label. Suitable fluorescent labels are described above. The probe may be an oligonucleotide having at least 90% sequence identity to SEQ ID NO: 3. Forward primer and the reverse primer may anneal to a target HPV16 sequence. The method may further comprise amplifying the target HPV16 sequence, if present in the sample, by droplet digital PCR. The method may further comprise detecting a signal from the detectable label, wherein detection of the signal indicates the presence of the target HPV16 sequence. The method may further comprise quantifying the amount of the target HPV16 present in the sample.

The cell free DNA may be isolated from plasma obtained from the subject. For example, plasma may be obtained and cell free DNA may be isolated using any suitable method. Cell free DNA may be isolated using a commercially available kit.

The method further involves modulating therapy in the subject based upon HPV16 ctDNA levels. The method may comprise increasing the dose and/or frequency of the therapy and/or providing an alternative or additional therapy to the subject when the change from baseline to the follow on level is above a threshold value. For example, the method may comprise increasing the dose and/or frequency of the therapy that has already been provided to the subject (e.g. the therapy provided to the subject prior to measuring the follow on level of HPV16 ctDNA). For example, the method may comprise increasing the frequency of therapy. As another example, the method may comprise increasing the dose of therapy. Increasing the dose of therapy may refer to increasing the amount given per individual dose and/or increasing the total number of doses given to the subject. The method may comprise providing an alternative therapy to the subject. For example, the method may comprise switching from the first form of therapy to a second, different form a therapy. The method may comprise providing an additional therapy to the subject.

The threshold value may be 50%. The threshold value may be 60%. For example, the method may comprise increasing the dose and/or frequency of the therapy and/or providing an alternative or additional therapy to the subject when the change from the baseline level to the follow on level is above 50% (e.g. at least 50%, at least 55% ,at least 60%, at least 65%, at least 70%, at least 75%, etc.).

## C. Oligonucleotides

In some aspects, provided herein is a set of oligonucleotides as defined in the claims. The set of oligonucleotides may be used in a method of amplifying and detecting a target HPV16 sequence, such as a target HPV16 ctDNA sequence. The set of oligonucleotides comprises a forward primer having at least 90% sequence identity (e.g. at least 90%, at least 91%, at least 92%, at least 93%, at least 94%, at least 95%, at least 96%, at least 97%, at least 98%, at least 99%, or 100%) to SEQ ID NO: 1, a reverse primer comprising SEQ ID NO: 2, and a probe having at least 90% sequence identity (e.g. at least 90%, at least 91%, at least 92%, at least 93%, at least 94%, at least 95%, at least 96%, at least 97%, at least 98%, at least 99%, or 100%) to SEQ ID NO: 3.

In some embodiments, the forward primer comprises the nucleotide sequence of SEQ ID NO: 1. The reverse primer comprises the nucleotide sequence of SEQ ID NO: 2. In some embodiments, the probe comprises the nucleotide sequence of SEQ ID NO: 3. In some embodiments, the forward primer comprises the nucleotide sequence of SEQ ID NO: 1, the reverse primer comprises the nucleotide sequence of SEQ ID NO: 2, and the probe comprises the nucleotide sequence of SEQ ID NO: 3. In some embodiments, the probe comprises a detectable label. In some embodiments, the probe comprises a fluorescent label. Suitable fluorescent labels include, for example, FAM (5- or 6-carboxyfluorescein), VIC, NED, Fluorescein, FITC, IRD-700/800, CY3, CY5, CY3.5, CY5.5, HEX, TET, TAMRA, JOE, ROX, BODIPY TMR, Oregon Green, Rhodamine Green, Rhodamine Red, Texas Red, Yakima Yellow, Alexa Fluor PET, Biosearch Blue™, Marina Blue®, Bothell Blue®, Alexa Fluor®, 350 FAM™, SYBR® Green 1, Fluorescein, EvaGreen™, Alexa Fluor® 488 JOE™, VIC™ HEX™ TET™, CAL Fluor® Gold 540, Yakima Yellow®, ROX™, CAL Fluor® Red 610, Cy3.5™, Texas Red®, Alexa Fluor® 0.568 Cy5™, Quasar™ 670, LightCycler Red640®, Alexa Fluor 633 Quasar™ 705, LightCycler Red705®, Alexa Fluor® 680, SYTO® 9, LC Green®, LC Green® Plus+, and EvaGreen™.

The set of oligonucleotides may generate an amplicon comprising SEQ ID NO: 4 or a portion thereof comprising at least 15 contiguous bases of SEQ ID NO:4. For example, the set of oligonucleotides comprise a forward primer and a reverse primer that generates an amplicon comprising SEQ ID NO: 4 or a portion thereof, and a probe that hybridizes to the amplicon.

### D. Kits

In some aspects, provided herein are kits. The kits may comprise a set of oligonucleotides, as described herein. The kits may further comprise reagents necessary to purify, isolate, detect and/or quantify HPV16 ctDNA. For example, the kits may further comprise PCR reagents, including buffers and enzymes (e.g. dUTPs, dNTPs). The kit may further comprise control samples, if needed. The kit may comprise solid surfaces (e.g., beads) comprising capture reagents (e.g., oligonucleotides) specific for target ctDNA. The kit can further include containers for holding or storing a sample (e.g., a container or cartridge for a plasma sample, a container for a cell free DNA sample, etc.). The kit can also include one or more instrument for assisting with obtaining a test sample, such as a syringe. Where appropriate, the kit optionally also can contain reaction vessels, mixing vessels, and other components that facilitate the preparation of reagents (e.g. a container for mixing reagents for PCR).

The kit may further comprise instructions for use of the kit. Instructions included in kits can be affixed to packaging material or can be included as a package insert, or can be viewed or downloaded from a particular website that is recited as part of the kit packaging or inserted materials. While the instructions are typically written or printed materials they are not limited to such. Any medium capable of storing such instructions and communicating them to an end user is contemplated by this disclosure. Such media include, but are not limited to, electronic storage media (e.g., magnetic discs, tapes, cartridges, chips), optical media (e.g., CD ROM), and the like. As used herein, the term "instructions" can include the address of an internet site that provides the instructions.

### EXAMPLES

### Example 1

### Results

Assay **Development and Analytical Validation**

HPV16 ctDNA Assay Optimization

To develop a novel HPV16 ctDNA assay, regions of the HPV16 genome that are retained throughout the course of HPV+ HNSCC cancer progression were characterized. A detailed characterization of the complete HPV genome in tumor metastases using a focused enrichment analysis has not previously been accomplished using sequencing-based approaches. Therefore, to provide comprehensive empirical evidence for regions of the HPV genome that are retained in metastatic lesions and could be the focus of the assay, targeted capture based sequencing of biopsy specimens collected from a prospective patient cohort was performed **(Figure 1A).** Results demonstrated that metastatic tumor genomes harbored genetic material from the upstream regulatory region (URR), E6/E7 and L1 loci, but also frequently lost genetic material that included a portion of E1, E2/E4, E8. Additionally, metastatic tumor A was also missing most of the L2 gene, supporting development of an assay within the L1 to E7 region of the genome.

In parallel, a bioinformatics-based approach was implemented to define local specificity of potential amplicon regions by comparing the HPV16 and HPV18 genomes. These two genomes were selected as they represent the highest and second highest HPV types in HPV+ cancers,[31] respectively. An 80 nucleotide sliding window was used to approximate the size of a small primer-probe amplicon-based assay (2 x 25nt primers + 30nt probe), given that most abundant cell free DNA expected to be accessible to standard ddPCR assay-design is in the range of 73-165nts.[32] This demonstrated that

the HPV16 and HPV18 genomes have minimal pairwise alignment at 80 nucleotide resolution with a few exceptions **(Figure 1B)**. Consequently assay design was focused within the minimal region of HPV16 DNA retained in both tumor genomes from Figure 1A, excluding the beginning of the E1 gene which had a relatively high pairwise alignment between HPV16 and HPV18.

For optimal primer and probe design, the assay was focused on a region of the HPV16 genome with GC-content around 45%, based on data showing the impact of GC-content on polymerase amplification ability of small amplicons.[33] Again using an 80nt sliding window, three potential regions of the focus region for assay design were identified **(Figure 1C),** and for initial design it was decided to focus on the region containing the end of the URR and beginning of E6 for the assay. Nine primer sets of varying lengths were designed to target this region with one assay focused on a 128 nucleotide amplicon and 8 assays closer to the minimal length of 83 to 77 nt. Each set of primers/probes was tested on control cell line genomic DNA by qPCR to test the relative sensitivity of HPV16 detection (from HPV16+ UM-SCC-104 genomic DNA) and confirm specificity over the HPV18 and HPV negative genomes (using HPV18+ UM-SCC-105 and HPV- UM-SCC-85), **Figure 1E.** This demonstrated a high sensitivity of the short amplicon assays relative to the long amplicon assay, and also confirmed the specificity of HPV16 detection. Three of the probe sets (V1, V5 and V9) were evaluated by ddPCR. As shown in the raindrop plots, assay V9 had the best signal-to-noise ratio and was therefore advanced for detailed analytical validation **(Figure 1F).**

Analytic Validation of the HPV16 ctDNA ddPCR assay

To determine the limit of detection for the assay, serial dilutions of synthetic HPV16 DNA containing the 77bp amplicon specific to assay V9 were used **(Figure 2A),** as well as serial dilutions of UM-SCC-104 cell line genomic DNA **(Figure 2B).** Through genome sequencing analysis, UM-SCC-104 has previously been demonstrated to have low copy number of a near complete HPV16 genome.[34] The synthetic HPV16 fragment was tested in the context of a background of normal human genomic DNA used as a carrier. Both UMSCC-104 cell line genomic DNA and normal human carrier DNA were digested with HindIII restriction enzyme, to better recapitulate the fragmented nature of plasma cfDNA. The LoD by both dilution series experiments was determined to be < 5 copies per reaction (see Materials and Methods) **(Figure 2A and 2B).** The ddPCR assay displayed a low variation among replicates at different concentrations as seen in the coefficient of variation (CV) plot of the restriction digested UMSCC-104 cell line **(Figure 2C),** which is derived from an HPV16+ HNSCC.

To then confirm specificity of the ddPCR assay, assay results on genomic DNA isolated from a cohort p16+ OPSCC tumors were compared to a series of melanoma tumor tissue specimens as HPV-negative controls. This confirmed the high specificity of the ddPCR assay for HPV16+ tumor tissue **(Figure 2C).** Finally, to further characterize the reproducibility of the overall assay process, multiple aliquots of plasma from a single patient over two time points (one with a low level of HPV16 ctDNA and the other which had a high level) were isolated independently and run separately with the assay. For the low-count sample, the mean count was 13 copies/uL with a standard deviation of 1.7 copies/uL. For the high-count sample, the mean count was 1978 copies/uL with a standard deviation of 71.3 copies/uL **(Figure 2D).** Collectively, this analytic data validated the high sensitivity and specificity of the HPV16 ddPCR assay.

**Clinical Performance of HPV ctDNA Assay**

Patient characteristics

Sixteen patients with p16 positive tumors were enrolled between October 2017 and April 2019 in which 102 distinct plasma sample were collected. The primers described herein were able to detect HPV16 ctDNA in baseline samples from 12 of these patients (12/16, 75%), all of whom had HPV type 16. Baseline characteristics of the 12 subjects are summarized in **Table 1.** Blood was collected with Paxgene tubes for all time points in 10 patients and Streck Cell-Free BCT tubes for all time points in 2 patients. Of these 12 subjects, 6 had ctDNA levels analyzed during two distinct treatment courses representing a total of 18 distinct treatment courses (85 distinct samples). There was an average of 5 samples collected per treatment regimen (range: 2-17). Other than one patient missed plasma collection on Cycle 2, Day 1, there were no missing samples. Sample collection is outlined in **Figures 3A and 5A.** Seven patients were treated with immunotherapy-containing regimens, while 11 treatment courses utilized cytotoxic chemotherapy. Eleven patients had restaging imaging after two cycles whereas 7 patients had imaging after 3 cycles. The average duration from initiation of therapy (baseline plasma collection) to radiographic restaging imaging was 70 days (range: 33-166). The average duration of one cycle of treatment (baseline plasma collection to cycle 2 plasma collection) was 27 days (range: 21-44) and the average time from cycle 2 plasma collection to restaging scans was 43 days (range: 10-138) **(Table 1).**

**Table 1. Patient demographics and treatment characteristics**

| Patient Demographics (n=12) | |
|---|---|
| **Sex** | |
| Male, N (%) | 9 (75%) |
| Female, N (%) | 3 (25%) |
| Age, years, Mean (SD) | 62 (7.6) |
| **Primary Treatment Received** | |
| Chemoradiation, N (%) | 11 (92%) |
| Surgery with adjuvant treatment as indicated, N (%) | 1 (8%) |
| **Smoking Status** | |
| Current, N(%) | 1 (8%) |
| Former, N(%) | 3 (25%) |
| Never, N(%) | 8 (67%) |
| **Type of recurrence at R/M diagnosis** | |
| Unresectable locoregional recurrence, N(%) | 3 (25%) |
| Distant metastases, N(%) | 9 (75%) |
| Treatments Characteristics (n=18)[1] | |
| Chemotherapy, N(%) | 11 (61%) |
| Immunotherapy, N(%) | 7 (38%) |
| Timing of imaging and plasma collection | Average (range) |
| Restaging imaging after 2 cycles[2] | 11 |
| Restaging imaging after 3 cycles[3] | 7 |
| Baseline collection→ restaging scans | 70 d (35-166) |
| Baseline collection→ Cycle 2 Day 1 | 27 d (20-49) |
| Cycle 2, Day 1→ restaging imaging | 38 d (10-82) |

[1]: 6 of 12 subjects had ctDNA collected during two distinct treatment courses representing a total of 18 distinct treatment courses
[2]: Performed on Cycle 3, Day 1
[3]: Performed on Cycle 4, Day 1

Change in plasma HPV16 ctDNA levels correlates with radiographically-determined treatment response

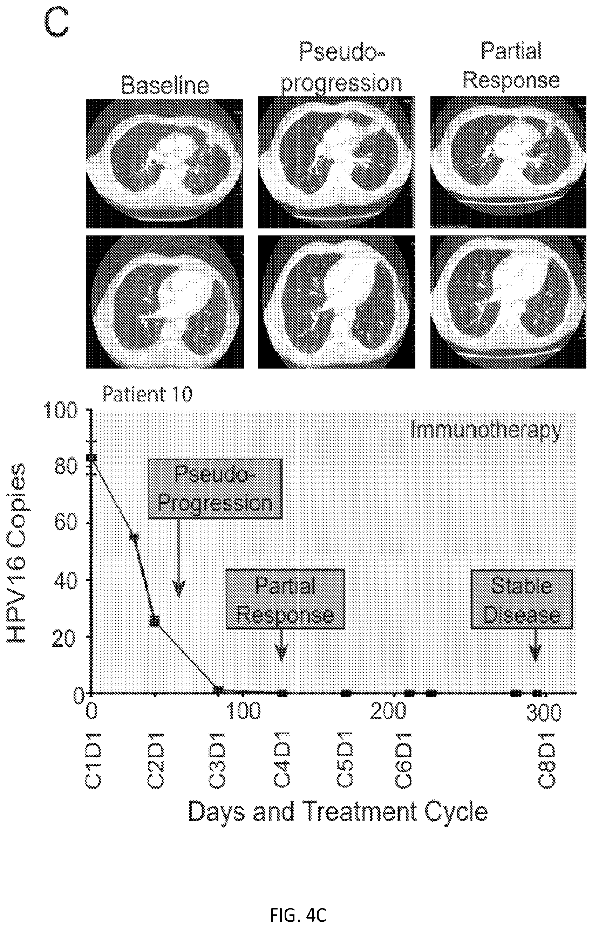

The concordance of detected radiographic progression was assessed at the time of standard of care re-imaging with an increase in HPV16 ctDNA observed in blood collected synchronously at the time of re-imaging. Thus, the change in HPV16 ctDNA from baseline to the time of restaging imaging at post-cycle 2 therapy or post-cycle 3 therapy time points was evaluated **(Figure 3A)**. Percent change in HPV16 ctDNA was associated with radiographic progression (**Figure 3B,** Wilcoxon p=0.02). Similarly, a relationship was observed for smaller increases (or larger decreases) in HPV16 ctDNA across response groups as defined by RECIST (PD, SD, PR, CR) (**Figure 3C,** Median analysis, p=0.01). ROC curve analysis optimizing Youden's index for prediction of progressive disease estimated a discriminatory cut point in the range of 30-60%. After evaluating the optimization in bootstrapped samples, $\geq 60\%$ increase was elected as a cut-point for prediction of progressive disease with optimal sensitivity and specificity. Dichotomizing patients into those with $\geq 60\%$ vs < 60% HPV16 ctDNA increase resulted in an AUC of 0.84 (p=0.02 **Figure 3D**), sensitivity of 88.9% (95% CI 52-99.7%), specificity of 88.9% (95% CI 52-99.7%), positive predictive value of 88.9% (95% CI 55-98.1%) and negative predictive value of 88.9% (95% CI 56-98.1%). Of subjects with <60% increase in HPV16 ctDNA, (8/9) had stable disease or partial response, and, of subjects with $\geq 60\%$ increase in HPV16 ctDNA (8/9) had progressive disease **(Figure 3E).**
Next, the longitudinal pattern of HPV16 ctDNA within each patient in relationship to their clinical history including treatment modality, symptoms of progression, and imaging findings was evaluated. A sample of patient HPV16 ctDNA levels and histories over time are shown in **Figure 4.** These graphs suggested that changes in HPV16 ctDNA levels occur prior to determination of radiographic response and an increase in ctDNA level precedes radiographic failures. This is remarkably shown in the case of Patient 1 **(Figure 4A)** in whom molecular progression was observed more than 100 days prior to detection of radiographic progression. Of particular interest were two cases of patients treated with immunotherapy in whom radiographic pseudo-progression was observed. In both cases, HPV16 ctDNA levels decreased, consistent with a molecular response despite suggestion of progressive disease on imaging **(Figure 3C).** Pseudoprogression has been described as an uncommon event occurring in less than 10% of patients treated with immunotherapy.[36] Patient 3 (**Figure 4B**) experienced potential symptoms of progression and initial response imaging was interpreted as progressive disease. Synchronous HPV16 ctDNA analysis noted a biochemical response with a 63% decrease. Treatment with immunotherapy was continued and the patient developed a partial response on follow up imaging which is ongoing for greater than 18 months. In a similar scenario, Patient 10 (depicted in **Figure 4C)** was treated with immunotherapy and the first set of restaging imaging demonstrated progressive disease as judged by RECIST. Given the possibility of pseudoprogression, he was continued on therapy and eventually achieved a durable near complete radiographic response until his death (unrelated to disease progression). HPV16 ctDNA analysis showed a marked decrease at the synchronous blood collection with 100% clearance of HPV16 ctDNA. Representative radiographic images from two different planes are shown in Figure 4C.

Given these observations, it was assessed if early changes in HPV16 ctDNA were predictive of future radiographic response to therapy. To test this, the change between baseline HPV16 ctDNA and that drawn prior to cycle 2 was investigated **(Figure 5A).** Sixteen subjects had available measurements after cycle 1 prior to cycle 2. The median (min-max) number of days prior to imaging was 33 (10-82). A significant association between change in HPV16 ctDNA at cycle 2 and later evidence of progression was found (**Figure 5B,** Wilcoxon p=0.02). A relationship was similarly observed for changes in HPV16 ctDNA across RECIST response categories which was statistically significant (**Figure 5C,** Kruskal-Wallis p=0.04). There was strong positive correlation between percent change seen in the draw prior to cycle 2 to that drawn synchronous to re-staging imaging (**Figure 5D,** Pearson rho=0.90).

## Discussion

Described herein is the development and detailed analytical validation of a high-precision ddPCR assay to quantify plasma HPV16 ctDNA, and the demonstration through a prospective study that results of radiographic assessment and simultaneously collected HPV16 ctDNA were highly concordant. Furthermore, analysis suggests change in HPV16 ctDNA after just one cycle of treatment is predictive of radiographic progression. Specifically, the data suggests that a <60% increase (or any decrease) in HPV16 ctDNA is associated with a favorable response to therapy (complete response, partial response, or stable disease), whereas patients with ≥60% increase in HPV16 ctDNA have disease progression and presumably do not derive any benefit from therapy. Finally, these data demonstrate that the percent change in HPV16 ctDNA after a single cycle of treatment correlates with that obtained synchronous to re-staging imaging.

Despite introduction of immunotherapeutic agents, survival for patients with R/M HNSCC remains poor.[6] Initial response to therapy can have a significant favorable impact on survival for some patients,[37] however a clear understanding of which patients will respond to each therapy is lacking. Additionally, futile systemic treatments can have significant toxic side effects. Currently established tissue biomarkers, including PD-L1, could theoretically be used to guide initial treatment selection based on historical outcomes but require invasive biopsy. In OPSCC, biopsies are often challenging due to the nature of the anatomy involved. Circulating biomarkers, on the other hand, allow for dynamic quantification of real-time treatment response obtained via minimally invasive approaches. The use of plasma ctDNA to guide treatment decisions is increasing in the oncology clinical setting.[38] For example, PCR-based ctDNA assays for EGFR genotyping in non-small cell lung cancer, and for KRAS genotyping in colorectal cancer have demonstrated clinical validity and thus have received regulatory approval in the United States and Europe.[39-41] Little investigation has been performed to date in R/M HPV+ OPSCC. This work demonstrated that patients with poor outcomes had higher plasma levels of ctDNA and that patients who responded to treatment were seen to have decrease in plasma HPV16 ctDNA levels whereas patients with progressive disease had increases in plasma HPV16 ctDNA.

The data demonstrate a role for HPV16 ctDNA for early detection of progressive disease in R/M HPV+ OPSCC. Accordingly, this assay finds use in guiding treatment decisions regarding efficacy of therapy, thereby potentially switching patients from futile therapy to more efficacious therapy more promptly. Furthermore, HPV16 ctDNA serves as a tool to distinguish clinical benefit and progressive disease on imaging in patients treated with immunotherapy given the phenomenon of pseudo-progression. The assay herein allows for a paradigm-defining approach to head and neck cancer in which systemic therapy is switched after one cycle based on biomarker indications of treatment failure, rather than delaying that intervention until radiographic imaging demonstrates disease progression. This data demonstrates that changes in HPV16 ctDNA is predictive of progressive disease in patients with R/M HNSCC receiving systemic therapy. Changes in HPV16 ctDNA precede radiographic response and thus find use as an early predictive biomarker to guide treatment decisions, improving survival and sparing toxicity.

## Materials and Methods

## Development of HPV ctDNA Assay

Droplet digital PCR (ddPCR) assay for HPV16 ctDNA

A Taqman probe-based ddPCR assay for the most common HPV subtype, HPV16, was developed as described below. Each ddPCR sample reaction contained 7uL of DNA with 1 uL HPV target assay mix (forward and reverse primers and TaqMan FAM probe), 1 uL RPP30 assay mix (with HEX probe, Assay ID: dHsaCP2500350), 1 uL nuclease free water, and 10 uL ddPCR Super Mix (no dUTP) (#186-3024, Bio-Rad, Hercules, CA). A ddPCR assay for the RPP30 control gene was used to assess sample quality (Bio-Rad, Hercules, CA). Each sample assay was run in duplicate. UM-SCC-104 (HPV16 positive) and UM-SCC-105 (HPV18 positive) cell line DNA were run to serve as positive and negative controls.

Droplet generation was performed using the QX200 Droplet Digital PCR System (Bio-Rad, Hercules, CA). PCR was run with the following thermocycler conditions: enzyme activation for 10 minutes at 95°C for 1 cycle, denaturation at 94°C for 30 seconds and annealing/extension at 60°C for 60 seconds for 40 cycles at a 2°C/second ramp rate, enzyme deactivation for 10 minutes at 98°C for 1 cycle, followed by a hold at 4°C. Droplets were read using QuantaSoft Software (Bio-Rad, Hercules, CA). Standardized thresholds for positive and negative droplets were used across samples. Samples were considered positive if they had 2 or more positive droplets at the same amplitude as the positive cell line control. The Bio-Rad system quantifies DNA in copies per uL of the 20 uL PCR reaction, which are the values reported here.

Calculation of Limit of Detection and Coefficient of Variation

To determine the Limit of detection (LoD), a synthetic 87 bp dsDNA fragment containing the 77 bp *HPV16 E6* amplicon from assay V9 (5' AATGCCGAAACCGGTTAGTATAAAAGC AGACATTTTATGCACCAAAAGAGAACTGCAA TGTTTCAGGACCCACAGGAGCGACT CACT 3') (SEQ ID NO: 15) or genomic DNA from UM-SCC-104 cell line was tested in a background human genomic DNA matrix (200,000 genome equivalents (GEs) per 20 μl ddPCR reaction). The sequence GCACCAAAAGAGAACTGCAATGTTTCAGGACCCACAGGAGCGAC (SEQ ID NO: 4) is the 77bp amplicon. In order to generate background matrix DNA, human genomic DNA (#PR-G3041, Promega) was incubated with 1 unit HindIII enzyme (# R0104S, New England Biolabs) per microgram of DNA at 37°C for 4 hours followed by enzyme inactivation at 80°C for 20 min. A 2-fold dilution series (2000 copies or GEs per μl to 4 copies or GEs per ul) was tested in triplicates using ddPCR and LoD was calculated using the formula:

$$\text{LoD} = \text{Limit of Blank (LoB)} + 1.645 \left(\text{Standard Deviation}_{\text{low concentration sample}}\right)$$

LoB was determined per 20 uL reaction using 200,000 diploid human GEs (HindIII digested) as a non-HPV background matrix (n=15). With UM-SCC-104 cell line, LoD = 0.3 copies/20 μl reaction based on standard deviation of 0.2 for the lowest concentration sample (4 GEs /μl); and with synthetic DNA (containing the 77bp amplicon sequence), LoD = 4.2 copies/20 μl reaction based on standard deviation of 2.54 for the lowest concentration sample (4 copies/μl). The Coefficient of Variation (CV) was calculated using the formula % CV = (Standard Deviation / Mean)*100.

Characterizing the precision of the HPV16 ctDNA ddPCR assay

One HPV16 ctDNA sample with low counts and one sample with high counts were selected to run reproducibility testing. ctDNA was extracted from 3 x 2 mL aliquots and each diluted 1:40 as described below. From this, 12 reaction assays were created for each sample. Droplet generation, PCR amplification, and droplet reading were performed as indicated above. HPV16 ctDNA counts were plotted. Means with standard deviations were calculated for the low and high sample. Standard curves were created to determine expected standard deviations for any given HPV16 ctDNA count.

HPV16 Genome Specificity Analysis

The genomic sequence for HPV16 (K02718.1) and HPV18 (AY262282.1) were downloaded from Genbank. One nucleotide-offset sliding windows of 18, 24, 34 and 77 nt were extracted from the HPV16 reference sequence, corresponding to the lengths of the reverse primer, forward primer, probe and amplicon sequence, respectively. Pairwise alignment scores were calculated between each of the HPV16 k-mer window sequences and the HPV18 genome using the pairwise alignment function (R package, Biostrings v2.52.0) with default settings for the "global-local" alignment type and scoreOnly=TRUE, which returns the maximum alignment score for each k-mer window sequence. The maximum alignment score for each k-mer window was plotted based on the start position of each window. Using the same settings, pairwise alignment scores were also calculated between the HPV18 genome and the HPV16 primer, probe and amplicon sequences, with 0-4 mutations (substituting "N" bases) introduced at random locations. Plots were generated using the R packages, ggplot2 (v3.2.1), cowplot (v1.0.0) and ggforce (v0.3.2).

## Evaluation of Assay Performance in Clinical Cohort

### Prospective Cohort

A prospective cohort was enrolled as part of a University of Michigan Institutional Review Board-approved, observational longitudinal biospecimen collection study (UM IRB 00042189). All patients provided written informed consent. Patients ≥ 18 years old with histologically documented HPV+ OPSCC undergoing systemic therapy were eligible. p16 was determined by tumor immunohistochemistry and was used as a surrogate marker for HPV status of a patient's cancer. All patients were required to have the presence of measurable disease by CT scan, or cutaneous lesions ≥ 10 mm not assessable on imaging but present on physical exam. Adequate hematopoietic and renal function were required and defined as hemoglobin ≥ 9.0 g/dL and serum.

Enrolled patients had baseline collection of clinical data on demographics, disease characteristics, treatment plan, laboratory studies (CBC with differential, comprehensive metabolic profile) and radiographic staging studies. Pre-treatment tumor tissue was obtained for next generation sequencing, whenever available. Duration of a treatment cycle was defined based on clinical documentation. Blood was collected at each visit during the treatment course as well as synchronous with radiographic imaging until disease progression, patient withdrawal of consent, or at investigator discretion. 10 mL of blood was collected from patients in sterile PreAnalytix Paxgene tubes (PreAnalytiX GmbH, Switzerland) or Streck Cell-Free DNA BCT tubes (Streck Inc., Omaha, NE, USA) with the same type of tube used for serial collection within a given participant. Previous publications of ctDNA analysis have demonstrated equivalence between these collection tubes.[27] Tubes were held at room temperature and processed as per manufacturer's recommendations within 7 days of collections as described in detail below. Re-staging imaging (after 2 or 3 cycles) was obtained at the frequency as determined by the discretion of the treating physician. Timing of the imaging and corresponding results were recorded in the database. Radiologic response was assessed according to RECIST v1.1. In the case of treatment continued despite RECIST-assessed progressive disease, due to suspected pseudoprogression, response was confirmed on subsequent scans. If a patient experienced progression and was started on a subsequent therapy, they were re-enrolled with identical approach of data and specimen collection.

### DNA isolation from Tumor Tissue

Two tumor tissue blocks from patients with R/M HPV+ OPSCC from the prospective cohort were available with sufficient material for NGS and eight blocks from primary HPV+ OPSCC and 7 with melanoma blocks were available with sufficient material for ddPCR based tumor analysis. DNA extraction from FFPE specimens was performed as previously described. [1, 2] Areas of tumor were identified by a board-certified head and neck pathologist (J.B.M). Tumor and adjacent normal tissue cores were collected and genomic DNA was obtained using a Qiagen Allprep DNA/RNA FFPE kit (Qiagen, Hilden, Germany) and quantified using Qubit as previously described. [2] For ddPCR assays, RPP30 Bio-Rad assay was used to measure RPP30 reference gene and ensure ample DNA being assessed in the case of HPV positive and HPV negative tumors (data not shown).

### Targeted next-generation sequencing to define HPV16 tumor DNA content

DNA from tumor tissue was submitted to the University of Michigan Advanced Genomics Core for targeted capture sequencing using the DNA Thruplex kit (Takara Biosciences). Targeted capture was performed using a custom designed probe panel from Nextera with high density coverage of HPV16 which is described in O'Leary et. al 2020.[3] Following library preparation and capture, the samples were sequenced on an Illumina HiSEQ4000, respectively, with paired-end 150nt reads. Data was de-multiplexed and FastQ files were generated. Samtools depth (samtools/1.9) was used to compute the read depth at each position across the HPV16 genome. The input is a BAM file derived from HPViewer with short reads aligned to the HPV genome. The unmapped, secondary, QC-fail and duplicate reads were skipped from the analysis. Only reads with base quality greater than 5 and mapping quality greater than 20 were counted, plots were created with Excel.

### DNA Extraction from Plasma

Plasma was isolated in two sequential centrifugation steps per manufacturer recommendations. Briefly, PaxGene tubes were centrifuged at 1900xg for 15 minutes; plasma layer was transferred into a 15mL conical tube and centrifuged a second time again at 1900xg for 10 minutes. Streck tubes were centrifuged at 300xg for 20 minutes; plasma layer was transferred into a 15 mL conical tube and centrifuged a second time at 4600xg (maximum possible speed) for 10 minutes. Cell free DNA was isolated from a 2 mL aliquot of plasma using QIAamp® MiniElute® ccfDNA Mini Kit (Qiagen, Germantown, MD) and diluted 1:40 according to manufacturer instructions. Genomic DNA from cell lines UM-SCC-104 (HPV16 positive), UM-SCC-105 (HPV18 positive), and UM-SCC-85 (HPV negative)[28] was extracted using the Promega Wizard DNA Purification Kit protocol (Promega A1120, Madison WI). Cell line DNA was used for assay optimization and as positive controls for plasma ctDNA assays.

Statistical analyses

The primary aim of the prospective pilot cohort was to assess the HPV16 ctDNA assay in a diverse patient population and assess for 1) concordance with imaging results and 2) potential signal of association of early dynamics with radiographic progression. Treatment responses, as determined by radiology, after 2-3 cycles of treatment, based on treatment protocol, were collected from the medical record. For each patient, HPV16 ctDNA counts/mL were plotted over time along with treatment histories. Percent change in HPV16 ctDNA level was calculated relative to a patient's own baseline ((time point level - baseline level)/baseline level) and assessed at two clinical episodes. The HPV16 ctDNA level closest to the radiographic assessment was chosen to test association of HPV16 ctDNA with synchronous imaging results whereas the level on day 1 of cycle 2 of therapy was chosen to explore the ability of HPV16 ctDNA to predict radiographic progression before the standard timeline of assessment. Percent change in HPV16 ctDNA across radiographic response groups were compared with nonparametric Wilcoxon, Kruskal-Wallis, and median one-way analysis tests. ROC curve analysis was performed for the prediction of progressive disease at time of imaging. From the ROC curve, area under the curves were extracted (AUC) as well as the optimal cutpoint using Youden index to maximize the sum of sensitivity and specificity for predicting progression. The optimal cutpoint was estimated from the full sample and in n=500 bootstrapped samples. Statistical analysis was performed using SPSS (v27) and R packages, ggplot2 (v3.2.1), cutpointR(v1.1.0).

**Abbreviations:**

HPV+: human papillomavirus related

OPSCC: oropharyngeal squamous cell carcinoma

ddPCR: droplet digital PCR

ctDNA: circulating tumor DNA

HNSCC: Head and neck squamous cell carcinomas

CV: coefficient of variation

**Example 2**

For HPV+ OPSCC, a large number of clinical trials are attempting to advance new treatment de-escalation strategies that maintain excellent survival outcomes while minimizing long-term toxicities.[2-6] However, these trials carry the risk of de-escalating patients that would have otherwise benefited from standard treatment; and, the identification of such patients, with ctDNA or other biomarkers, remains of paramount importance. Retrospective data with small patient cohorts suggest that HPV ctDNA is difficult to detect in patients with early stage disease (26% of patients with T1, 48% of patients with T2 disease)[7,8], levels correlate with disease burden,[9,10] rapid clearance after chemoradiation (CRT) is associated with improved locoregional control[11], and levels increase at the time of recurrence[9,12]. Importantly, very limited data has been published from low risk OPSCC patients and the published studies have few failures, preventing the rational design of HPV ctDNA trials in this setting.

A phase II clinical trial of low-risk HPV+ OPSCC was completed to determine whether treatment stratification by neck dissection, to more accurately pathologically stage patients, could minimize treatment modalities and thereby improve quality of life. The trial cohort therefore provided the unique opportunity to prospectively evaluate HPV ctDNA clearance following various modes of therapy in low-risk disease and to test the utility of HPV ctDNA for early detection of recurrence during surveillance.

Twenty-seven T1 and T2 patients with limited neck disease had plasma drawn before treatment and, 35.7% (5/14) of T1, and 69%(9/13) of T2 patients had detectable HPV ctDNA at baseline. Baseline HPV ctDNA levels were significantly higher in patients with T2 disease compared with T1 disease (averages of 2,467 vs 130.7 copies per milliliter plasma, respectively, *P<0.05)*. There were no differences in baseline plasma HPV ctDNA counts based on N classification or presence of extracapsular extension. All patients had complete clearance of plasma HPV ctDNA at 4 weeks post treatment regardless of modality **(Figure 6)**.

In total, 81 post-treatment plasma specimens from patients in this cohort were evaluated with a median follow up duration of 33.3 months (range: 0.56-39.6 months). One patient had detectable ctDNA at baseline that became undetectable 4 weeks post-surgery. Then, HPV ctDNA was detected six months post-treatment and persisted through the next 6 time points **(Figure 7)**. Importantly, 24 months after treatment (18 months after initial biochemical recurrence) a local recurrence was clinically identified. All post-treatment samples from other patients had no detectable biochemical recurrence and no other patient recurred clinically/radiologically.

Ongoing de-escalation strategies use various modalities to allow for de-intensified adjuvant treatment[3-6]. While this trial utilized the pathologic findings from neck dissection as a clinical biomarker to guide primary treatment modality, leveraging the cohort to gain insight into the clearance of HPV ctDNA in early stage disease was possible. The data demonstrate that all patients had undetectable plasma HPV ctDNA within one month of completion of treatment regardless of treatment modality and future recurrence. However, one of these patients developed recurrent disease, in spite of undetectable HPV ctDNA in plasma. These data are consistent with current working mathematical models of ctDNA shedding,[13] which suggest that tumor size, death and other shedding characteristics may limit the amount of ctDNA in circulation to below detectable levels with current technology. Despite this limitation, recent published data in a variety of settings suggest that plasma HPV ctDNA may be a complementary biomarker for identification of residual or recurrent disease.[7-9,11,12] Herein, data is provided showing that HPV ctDNA was detectable 18 months prior to overt clinical or radiographic recurrence.

**Methods**

Thirty-four patients with low-risk HPV+ OPSCC (T1-3, N0-N1) were enrolled (NCT02784288). Patients underwent upfront neck dissection with stratification into one of three treatment groups based on post-operative pathology. Patients with a single lymph node < 6 cm with no extracapsular extension (ECE), perineural (PNI), or perivascular invasion (PVI) underwent transoral surgery. Patients with two or more positive nodes without adverse features (ECE or positive margins) or a single node in which PVI or PNI was noted were treated with radiotherapy (RT) alone. Patients with ECE underwent CRT. Plasma was isolated from blood collected in Streck tubes from 27 patients prior to the neck dissection, one-month post-treatment (surgery, RT, or CRT), and every 3 months thereafter.

CfDNA was isolated following the QIAamp MinElute ccfDNA Mini Kit protocol. A digital PCR ctDNA assay was performed to amplify a 77bp region of HPV16_E6 with forward 5'-CGAAACCGGTTAGTATAAAAGCAG-3', reverse 5'-GTCGCTCCTGTGGGTCCT-3' and probe 5'-FAM-CATTTTATGCACCAAAAGAGAACTGCAATGTTTC-MGBNFQ-3'. Each reaction assay contained 10 μL of 2x dPCR Supermix for Probes (No dUTP), 0.9 μmol/L of respective primers, 0.25 μmol/L of respective probes, and 5 to 50 ng of cfDNA in a final volume of 20 μL. dPCR assays were performed on the QX100 and/or QX200 platforms outfitted with an automated droplet generator (Bio-Rad). An *RPP30*-HEX reference was used to assess quality (Assay-ID: dHsaCP2500350, Bio-Rad).

**References**

1 Chatfield-Reed K, Roche VP, Pan Q. cfDNA detection for HPV+ squamous cell carcinomas. Oral Oncol 2021;115:104958. https://doi.org/10.1016/j.oraloncology.2020.104958.

2 Machtay M, Moughan J, Trotti A, Garden AS, Weber RS, Cooper JS, et al. Factors Associated With Severe Late Toxicity After Concurrent Chemoradiation for Locally Advanced Head and Neck Cancer: An RTOG Analysis. JCO 2008;26:3582-9. https://doi.org/10.1200/JCO.2007.14.8841.

3 Chera BS, Amdur RJ, Green R, Shen C, Gupta G, Tan X, et al. Phase II Trial of De-Intensified Chemoradiotherapy for Human Papillomavirus-Associated Oropharyngeal Squamous Cell Carcinoma. JCO 2019;37:2661-9. https://doi.org/10.1200/JCO.19.01007.

4 Chen AM, Felix C, Wang P-C, Hsu S, Basehart V, Garst J, et al. Reduced-dose radiotherapy for human papillomavirus-associated squamous-cell carcinoma of the oropharynx: a single-arm, phase 2 study. The Lancet Oncology 2017;18:803-11. https://doi.org/10.1016/S1470-2045(17)30246-2.

5 Ferris RL, Flamand Y, Weinstein GS, Li S, Quon H, Mehra R, et al. Transoral robotic surgical resection followed by randomization to low- or standard-dose IMRT in resectable p16+ locally advanced oropharynx cancer: A trial of the ECOG-ACRIN Cancer Research Group (E3311). JCO 2020;38:6500-6500. https://doi.org/10.1200/JCO.2020.38.15_suppl.6500.

6 Nichols AC, Theurer J, Prisman E, Read N, Berthelet E, Tran E, et al. Radiotherapy versus transoral robotic surgery and neck dissection for oropharyngeal squamous cell carcinoma (ORATOR): an open-label, phase 2, randomised trial. The Lancet Oncology 2019;20:1349-59. https://doi.org/10.1016/S1470-2045(19)30410-3.

7 Ahn SM, Chan JYK, Zhang Z, Wang H, Khan Z, Bishop JA, et al. Saliva and plasma quantitative polymerase chain reaction-based detection and surveillance of human papillomavirus-related head and neck cancer. JAMA Otolaryngol Head Neck Surg 2014;140:846-54. https://doi.org/10.1001/jamaoto.2014.1338.

8 Dahlstrom KR, Li G, Hussey CS, Vo JT, Wei Q, Zhao C, et al. Circulating human papillomavirus DNA as a marker for disease extent and recurrence among patients with oropharyngeal cancer. Cancer 2015;121:3455-64. https://doi.org/10.1002/cncr.29538.

9 Hanna GJ, Supplee JG, Kuang Y, Mahmood U, Lau CJ, Haddad RI, et al. Plasma HPV cell-free DNA monitoring in advanced HPV-associated oropharyngeal cancer. Ann Oncol 2018;29:1980-6. https://doi.org/10.1093/annonc/mdy251.

10 Damerla RR, Lee NY, You D, Soni R, Shah R, Reyngold M, et al. Detection of Early Human Papillomavirus-Associated Cancers by Liquid Biopsy. JCO Precis Oncol 2019;3:. https://doi.org/10.1200/PO.18.00276.

11 Chera BS, Kumar S, Beaty BT, Marron D, Jefferys S, Green R, et al. Rapid Clearance Profile of Plasma Circulating Tumor HPV Type 16 DNA during Chemoradiotherapy Correlates with Disease Control in HPV-Associated Oropharyngeal Cancer. Clin Cancer Res 2019;25:4682-90. https://doi.org/10.1158/1078-0432.CCR-19-0211.

12 Chera BS, Kumar S, Shen C, Amdur R, Dagan R, Green R, et al. Plasma Circulating Tumor HPV DNA for the Surveillance of Cancer Recurrence in HPV-Associated Oropharyngeal Cancer. J Clin Oncol 2020;38:1050-8. https://doi.org/10.1200/JCO.19.02444.

13 Avanzini S, Kurtz DM, Chabon JJ, Moding EJ, Hori SS, Gambhir SS, et al. A mathematical model of ctDNA shedding predicts tumor detection size. Sci Adv 2020;6:. https://doi.org/10.1126/sciadv.abc4308.

14 Haring CT, Bhambani C, Brummel C, Jewell B, Bellile E, Heft Neal M, et al. Human Papilloma Virus Circulating Tumor DNA Assay Predicts Treatment Response in Recurrent/Metastatic Head and Neck Squamous Cell Carcinoma. Oncotarget n.d.;In Press**:**

## Claims

1. A method for detecting HPV16 circulating tumor DNA (ctDNA), the method comprising:

   a. contacting a sample comprising cell free DNA with a set of oligonucleotides comprising a forward primer having at least 90% sequence identity to SEQ ID NO: 1, a reverse primer comprising SEQ ID NO: 2; and a probe comprising a detectable label, wherein the forward primer and the reverse primer anneal to a target HPV16 sequence;
   b. amplifying the target HPV16 sequence, if present in the sample; and
   c. detecting a signal from the detectable label, wherein detection of the signal indicates the presence of the target HPV16 sequence in the sample.

2. The method of claim 1, wherein the probe is an oligonucleotide having at least 90% sequence identity to SEQ ID NO: 3.

3. The method of claim 1 or claim 2, wherein the target HPV16 sequence comprises the sequence of SEQ ID NO: 4 or a portion thereof comprising at least 15 continuous bases of SEQ ID NO:4.

4. The method of any of claims 1-3, wherein said method is further defined by at least one of the following:

   i) the probe comprises a fluorescent label;
   ii) amplifying the target HPV16 sequence is performed by quantitative PCR (qPCR),

wherein amplifying the qPCR is preferably digital PCR, wherein further preferably the digital PCR is droplet digital PCR;

iii) the cell free DNA is isolated from a plasma sample or from a saliva sample.

5. A method for quantifying HPV16 circulating tumor DNA (ctDNA), the method comprising:

a. contacting a sample comprising cell free DNA with a set of oligonucleotides comprising a forward primer having at least 90% sequence identity to SEQ ID NO: 1, a reverse primer comprising SEQ ID NO: 2; and a probe comprising a detectable label, wherein forward primer and the reverse primer anneal to a target HPV16 sequence;

b. amplifying the target HPV16 sequence, if present in the sample, by droplet digital PCR;

c. detecting a signal from the detectable label, wherein detection of the signal indicates the presence of the target HPV16 sequence; and

d. quantifying the amount of the target HPV16 sequence present in the sample.

6. The method of claim 5, wherein

i) the probe is an oligonucleotide having at least 90% sequence identity to SEQ ID NO: 3
and/or

ii) the forward primer comprises the nucleotide sequence of SEQ ID NO: 1, the reverse primer comprises the nucleotide sequence of SEQ ID NO: 2, and the probe comprises the nucleotide sequence of SEQ ID NO: 3.

7. The method of claim 5 or claim 6, wherein said method is further defined by at least one of the following:

i) the probe comprises a fluorescent label;

ii) the cell free DNA is isolated from a plasma sample or from a saliva sample.

8. A set of oligonucleotides for amplifying and detecting a target HPV16 sequence, the set of oligonucleotides comprising:

a. a forward primer having at least 90% sequence identity to SEQ ID NO: 1, wherein said forward primer preferably comprises the nucleotide sequence of SEQ ID NO: 1;

b. a reverse primer comprising the nucleotide sequence of SEQ ID NO: 2; and

c. a probe having at least 90% sequence identity to SEQ ID NO: 3, wherein said probe preferably comprises the nucleotide sequence of SEQ ID NO: 3.

9. The set of claim 8, wherein the probe comprises a detectable label, wherein the detectable label is preferably a fluorescent label.

10. A method of predicting response to treatment in a subject with HPV positive head and neck squamous cell carcinoma (HPV+ HNSCC), the method comprising:

a. measuring a baseline level HPV16 ctDNA in the subject;

b. measuring a follow on level of HPV16 ctDNA following one or more treatment sessions in the subject; and

c. predicting a positive response to treatment in the subject when a change from the baseline level to the follow on level is below a threshold value; or

d. predicting a negative response to treatment in the subject when a change from the baseline level to the follow on level is above a threshold value,

wherein measuring the baseline level and measuring the follow on level comprise:

(i) contacting a sample comprising cell free DNA from the subject with a set of oligonucleotides comprising a forward primer having at least 90% sequence identity to SEQ ID NO: 1, a reverse primer comprising SEQ ID NO: 2; and a probe comprising a detectable label, wherein the forward primer and the reverse primer anneal to a target HPV16 sequence;

(ii) amplifying the target HPV16 sequence, if present in the sample, by droplet digital PCR; and

(iii) detecting a signal from the detectable label, wherein the signal indicates the level of the target HPV16 sequence in the sample.

11. The method of claim 10, wherein the baseline level is measured prior to any treatment in the subject or the baseline level is measured following a first treatment cycle and the follow on level is measured following a second treatment cycle.

12. The method of claim 10, wherein said method is further defined by at least one of the following:

 i) the probe comprises a fluorescent label;
 ii) the probe is an oligonucleotide having at least 90% sequence identity to SEQ ID NO: 3;
 iii) the cell free DNA is isolated from a plasma sample obtained from the subject or from a saliva sample obtained from the subject;
 iv) the forward primer comprises the nucleotide sequence of SEQ ID NO: 1, the reverse primer comprises the nucleotide sequence of SEQ ID NO: 2, and the probe comprises the nucleotide sequence of SEQ ID NO: 3.

13. The method of any one of claims 10-12, wherein said method is further defined by at least one of the following:

 i) the threshold value is 50%;
 ii) the threshold value is 60%;
 iii) a positive response to treatment indicates a complete response to treatment, a partial response to treatment, or a stable disease state in the subject, or a negative response to treatment indicates disease progression in the subject;
 iv) the treatment is immunotherapy;
 v) the HNSCC is oropharyngeal squamous cell carcinoma (OPSCC).

**Patentansprüche**

1. Verfahren zum Nachweis von zirkulierender Tumor-DNA (ctDNA) von HPV16, das Verfahren umfassend:

 a. In-Kontakt-bringen einer Probe, die zellfreie DNA enthält, mit einem Satz von Oligonukleotiden, der einen Vorwärtsprimer mit mindestens 90 % Sequenzidentität zu SEQ ID NO: 1, einen Rückwärtsprimer, der SEQ ID NO: 2 umfasst, und eine Sonde, die eine nachweisbare Markierung umfasst, wobei der Vorwärtsprimer und der Rückwärtsprimer an eine Ziel-HPV16-Sequenz anlagern;
 b. Amplifizieren der Ziel-HPV16-Sequenz, wenn diese in der Probe vorhanden ist; und
 c. Detektieren eines Signals von der nachweisbaren Markierung, wobei das Detektieren des Signals das Vorhandensein der Ziel-HPV16-Sequenz in der Probe anzeigt.

2. Verfahren nach Anspruch 1, wobei die Sonde ein Oligonukleotid mit mindestens 90 % Sequenzidentität zu SEQ ID NO: 3 ist.

3. Verfahren nach Anspruch 1 oder Anspruch 2, wobei die Ziel-HPV16-Sequenz die Sequenz von SEQ ID NO: 4 oder einen Teil davon umfasst, der mindestens 15 aufeinanderfolgende Basen von SEQ ID NO: 4 umfasst.

4. Verfahren nach einem der Ansprüche 1 bis 3, wobei das Verfahren ferner durch mindestens eines der folgenden Merkmale definiert ist:

 i) die Sonde umfasst eine Fluoreszenzmarkierung;
 ii) die Amplifikation der Ziel-HPV16-Sequenz erfolgt mittels quantitativer PCR (qPCR), wobei die qPCR vorzugsweise eine digitale PCR ist, wobei die digitale PCR ferner vorzugsweise eine Tröpfchen-Digital-PCR ist;
 iii) die zellfreie DNA wird aus einer Plasmaprobe oder aus einer Speichelprobe isoliert.

5. Verfahren zur Quantifizierung von zirkulierender Tumor-DNA (ctDNA) von HPV16, das Verfahren umfassend:

 a. In-Kontakt-bringen einer Probe, die zellfreie DNA umfasst, mit einem Satz von Oligonukleotiden, der einen Vorwärtsprimer mit mindestens 90 % Sequenzidentität zu SEQ ID NO: 1, einen Rückwärtsprimer, der SEQ ID NO: 2 umfasst, und eine Sonde, die eine nachweisbare Markierung umfasst, wobei der Vorwärtsprimer und der Rückwärtsprimer an eine Ziel-HPV16-Sequenz anlagern;
 b. Amplifizieren der Ziel-HPV16-Sequenz, wenn diese in der Probe vorhanden, mittels digitaler Tröpfchen-PCR;
 c. Detektieren eines Signals der nachweisbaren Markierung, wobei das Detektieren des Signals das Vorhandensein der Ziel-HPV16-Sequenz anzeigt; und

d. Quantifizieren der Menge der in der Probe vorhandenen Ziel-HPV16-Sequenz.

**6.** Verfahren nach Anspruch 5, wobei

i) die Sonde ein Oligonukleotid mit einer Sequenzidentität von mindestens 90 % zu SEQ ID NO: 3 ist und/oder

ii) der Vorwärtsprimer die Nukleotidsequenz von SEQ ID NO: 1 umfasst, der Rückwärtsprimer die Nukleotidsequenz von SEQ ID NO: 2 umfasst und die Sonde die Nukleotidsequenz von SEQ ID NO: 3 umfasst.

**7.** Verfahren nach Anspruch 5 oder Anspruch 6, wobei das Verfahren ferner durch mindestens eines der folgenden Merkmale definiert ist:

i) die Sonde umfasst eine Fluoreszenzmarkierung;

ii) die zellfreie DNA wird aus einer Plasmaprobe oder aus einer Speichelprobe isoliert.

**8.** Ein Satz von Oligonukleotiden zur Amplifikation und zum Nachweis einer Ziel-HPV16-Sequenz, der Satz von Oligonukleotiden umfassend:

a. einen Vorwärtsprimer mit einer Sequenzidentität von mindestens 90 % zu SEQ ID NO: 1, wobei der Vorwärtsprimer vorzugsweise die Nukleotidsequenz von SEQ ID NO: 1 umfasst;

b. einen Rückwärtsprimer, der die Nukleotidsequenz von SEQ ID NO: 2 umfasst; und

c. eine Sonde mit mindestens 90 % Sequenzidentität zu SEQ ID NO: 3, wobei die Sonde vorzugsweise die Nukleotidsequenz von SEQ ID NO: 3 umfasst.

**9.** Der Satz nach Anspruch 8, wobei die Sonde eine nachweisbare Markierung umfasst, wobei die nachweisbare Markierung vorzugsweise eine fluoreszierende Markierung ist.

**10.** Verfahren zur Vorhersage des Ansprechens auf eine Behandlung bei einem Subjekt mit HPV-positivem Plattenepithelkarzinom im Kopf- und Halsbereich (HPV+ HNSCC), das Verfahren umfassend:

a. Messen eines Ausgangswerts für HPV16-ctDNA bei dem Subjekt;

b. Messen eines Folgewerts von HPV16-ctDNA nach einer oder mehreren Behandlungssitzungen bei dem Subjekt; und

c. Vorhersagen eines positiven Ansprechens auf die Behandlung bei dem Subjekt, wenn eine Veränderung vom Ausgangswert zum Folgewert unter einem Schwellenwert liegt; oder

d. Vorhersagen eines negativen Ansprechens auf die Behandlung bei dem Subjekt, wenn eine Veränderung vom Ausgangswert zum Folgewert über einem Schwellenwert liegt,

wobei das Messen des Ausgangswerts und das Messen des Folgewerts Folgendes umfassen:

(i) In-Kontakt-bringen einer Probe, die zellfreie DNA des Subjekts enthält, mit einem Satz von Oligonukleotiden, der einen Vorwärtsprimer mit mindestens 90 % Sequenzidentität zu SEQ ID NO: 1, einen Rückwärtsprimer, der SEQ ID NO: 2 umfasst, und eine Sonde, die eine nachweisbare Markierung umfasst, enthält, wobei der Vorwärtsprimer und der Rückwärtsprimer an eine Ziel-HPV16-Sequenz anlagern;

(ii) Amplifizieren der Ziel-HPV16-Sequenz, wenn diese in der Probe vorhanden ist, mittels digitaler Tröpfchen-PCR; und

(iii) Nachweisen eines Signals von der nachweisbaren Markierung, wobei das Signal den Gehalt der Ziel-HPV16-Sequenz in der Probe angibt.

**11.** Verfahren nach Anspruch 10, wobei der Ausgangswert vor einer Behandlung des Subjekts gemessen wird oder der Ausgangswert nach einem ersten Behandlungszyklus gemessen wird und der Folgewert nach einem zweiten Behandlungszyklus gemessen wird.

**12.** Verfahren nach Anspruch 10, wobei das Verfahren ferner durch mindestens eines der folgenden Merkmale definiert ist:

i) die Sonde umfasst eine fluoreszierende Markierung;

ii) die Sonde ist ein Oligonukleotid mit einer Sequenzidentität von mindestens 90 % zu SEQ ID NO: 3;
iii) die zellfreie DNA wird aus einer von dem Subjekt entnommenen Plasmaprobe oder aus einer von dem Subjekt erhaltenen Speichelprobe isoliert;
iv) der Vorwärtsprimer umfasst die Nukleotidsequenz von SEQ ID NO: 1, der Rückwärtsprimer umfasst die Nukleotidsequenz von SEQ ID NO: 2 und die Sonde umfasst die Nukleotidsequenz von SEQ ID NO: 3.

13. Verfahren nach einem der Ansprüche 10 bis 12, wobei das Verfahren ferner durch mindestens eines der folgenden Merkmale definiert ist:

i) der Schwellenwert beträgt 50 %;
ii) der Schwellenwert beträgt 60 %;
iii) ein positives Ansprechen auf die Behandlung indiziert ein vollständiges Ansprechen auf die Behandlung, ein partielles Ansprechen auf die Behandlung oder einen stabilen Krankheitszustand beim Subjekt, während ein negatives Ansprechen auf die Behandlung ein Fortschreiten der Erkrankung beim Subjekt indiziert;
iv) die Behandlung ist eine Immuntherapie;
v) das HNSCC ist ein oropharyngeales Plattenepithelkarzinom (OPSCC).

## Revendications

1. Procédé de détection d'ADN tumoral circulant HPV16 (ADNct), le procédé comprenant :

a. la mise en contact d'un échantillon comprenant de l'ADN acellulaire avec un ensemble d'oligonucléotides comprenant une amorce sens possédant au moins 90 % d'identité de séquence avec SEQ ID NO: 1, une amorce antisens comprenant SEQ ID NO: 2 ; et une sonde comprenant un marqueur détectable, dans lequel l'amorce sens et l'amorce antisens s'hybrident à une séquence HPV16 cible ;
b. l'amplification de la séquence cible HPV16, si elle est présente dans l'échantillon ; et
c. la détection d'un signal provenant du marqueur détectable, la détection du signal indiquant la présence de la séquence HPV16 cible dans l'échantillon.

2. Procédé selon la revendication 1, dans lequel la sonde est un oligonucléotide possédant au moins 90 % d'identité de séquence avec SEQ ID NO: 3.

3. Procédé selon la revendication 1 ou la revendication 2, dans lequel la séquence HPV16 cible comprend la séquence de SEQ ID NO: 4 ou une partie de celle-ci comprenant au moins 15 bases continues de SEQ ID NO:4.

4. Procédé selon l'une quelconque des revendications 1 à 3, dans lequel ledit procédé est en outre défini par au moins l'un des éléments suivants :

i) la sonde comprend un marqueur fluorescent ;
ii) l'amplification de la séquence HPV16 cible est effectuée par PCR quantitative (qPCR), l'amplification de qPCR étant de préférence une PCR numérique, encore préférablement la PCR numérique est une PCR numérique en gouttelettes ;
iii) l'ADN acellulaire est isolé à partir d'un échantillon de plasma ou à partir d'un échantillon de salive.

5. Procédé de quantification d'ADN tumoral circulant HPV16 (ADNct), le procédé comprenant :

a. la mise en contact d'un échantillon comprenant de l'ADN acellulaire avec un ensemble d'oligonucléotides comprenant une amorce sens possédant au moins 90 % d'identité de séquence avec SEQ ID NO: 1, une amorce antisens comprenant SEQ ID NO: 2 ; et une sonde comprenant un marqueur détectable, dans lequel l'amorce sens et l'amorce antisens s'hybrident à une séquence HPV16 cible ;
b. l'amplification de la séquence HPV16 cible, si elle est présente dans l'échantillon, par PCR numérique en gouttelettes ;
c. la détection d'un signal provenant du marqueur détectable, la détection du signal indiquant la présence de la séquence HPV16 cible ; et
d. la quantification de la quantité de la séquence HPV16 cible présente dans l'échantillon.

6. Procédé selon la revendication 5, dans lequel

i) la sonde est un oligonucléotide possédant au moins 90 % d'identité de séquence avec SEQ ID NO: 3 et/ou

ii) l'amorce sens comprend la séquence nucléotidique de SEQ ID NO: 1, l'amorce antisens comprend la séquence nucléotidique de SEQ ID NO: 2, et la sonde comprend la séquence nucléotidique de SEQ ID NO: 3.

7. Procédé selon la revendication 5 ou la revendication 6, dans lequel ledit procédé est en outre défini par au moins l'un des éléments suivants :

i) la sonde comprend un marqueur fluorescent ;

ii) l'ADN acellulaire est isolé à partir d'un échantillon de plasma ou à partir d'un échantillon de salive.

8. Ensemble d'oligonucléotides pour l'amplification et la détection d'une séquence HPV16 cible, l'ensemble d'oligonucléotides comprenant :

a. une amorce sens possédant au moins 90 % d'identité de séquence avec SEQ ID NO: 1, où ladite amorce sens comprend de préférence la séquence nucléotidique de SEQ ID NO: 1 ;

b. une amorce antisens comprenant la séquence nucléotidique de SEQ ID NO: 2 ; et

c. une sonde possédant au moins 90 % d'identité de séquence avec SEQ ID NO: 3, où ladite sonde comprend de préférence la séquence nucléotidique de SEQ ID NO: 3.

9. Ensemble selon la revendication 8, dans lequel la sonde comprend un marqueur détectable, dans lequel le marqueur détectable est de préférence un marqueur fluorescent.

10. Procédé de prédiction de la réponse à un traitement chez un sujet atteint d'un carcinome épidermoïde de la tête et du cou positif au HPV (HPV+ HNSCC), le procédé comprenant :

a. la mesure d'un niveau de base d'ADNct de HPV16 chez le sujet ;

b. la mesure d'un niveau de suivi d'ADNct de HPV16 après une ou plusieurs séances de traitement chez le sujet ; et

c. la prédiction d'une réponse positive au traitement chez le sujet lorsqu'un changement depuis le niveau de base jusqu'au niveau de suivi est inférieur à une valeur seuil ; ou

d. la prédiction d'une réponse négative au traitement chez le sujet lorsqu'un changement depuis le niveau de base jusqu'au niveau de suivi est supérieur à une valeur seuil,

la mesure du niveau de base et la mesure du niveau de suivi comprenant :

(i) la mise en contact d'un échantillon comprenant de l'ADN acellulaire du sujet avec un ensemble d'oligonucléotides comprenant une amorce sens possédant au moins 90 % d'identité de séquence avec SEQ ID NO: 1, une amorce antisens comprenant SEQ ID NO: 2 ; et une sonde comprenant un marqueur détectable, dans lequel l'amorce sens et l'amorce antisens s'hybrident à une séquence HPV16 cible ;

(ii) l'amplification de la séquence HPV16 cible, si elle est présente dans l'échantillon, par PCR numérique en gouttelettes ; et

(iii) la détection d'un signal provenant du marqueur détectable, le signal indiquant le niveau de la séquence HPV16 cible dans l'échantillon.

11. Procédé selon la revendication 10, dans lequel le niveau de base est mesuré avant tout traitement chez le sujet ou le niveau de base est mesuré après un premier cycle de traitement et le niveau de suivi est mesuré après un deuxième cycle de traitement.

12. Procédé selon la revendication 10, ledit procédé étant en outre défini par au moins l'un des éléments suivants :

i) la sonde comprend un marqueur fluorescent ;

ii) la sonde est un oligonucléotide possédant au moins 90 % d'identité de séquence avec SEQ ID NO: 3 ;

iii) l'ADN acellulaire est isolé à partir d'un échantillon de plasma obtenu du sujet ou à partir d'un échantillon de salive obtenu du sujet ;

iv) l'amorce sens comprend la séquence nucléotidique de SEQ ID NO: 1, l'amorce antisens comprend la séquence nucléotidique de SEQ ID NO: 2, et la sonde comprend la séquence nucléotidique de SEQ ID NO: 3.

**13.** Procédé selon l'une quelconque des revendications 10 à 12, dans lequel ledit procédé est en outre défini par au moins l'un des éléments suivants :

i) la valeur seuil est de 50 % ;
ii) la valeur seuil est de 60 % ;
iii) une réponse positive au traitement indique une réponse totale au traitement, une réponse partielle au traitement, ou un état pathologique stable chez le sujet, ou une réponse négative au traitement indique une progression de la maladie chez le sujet ;
iv) le traitement est une immunothérapie ;
v) le HNSCC est un carcinome épidermoïde oropharyngé (OPSCC) .

FIG. 1A-1C

| Transcript | Forward Primer | Reverse Primer | TaqMan Probe | Size |
|---|---|---|---|---|
| HPV16-E6 v1 | GAACCGAACCGGTTAGTAT AA (SEQ ID NO: 5) | ATGTATAGTTGTTTGCAGCTCT GT (SEQ ID NO: 13) | CATTTTATGCACCAAAAGAGAAC TGCAATGTTTC (SEQ ID NO: 3) | 128 |
| HPV16-E6 v2 | TTGAACCGAAACGGTTAGT (SEQ ID NO: 6) | GTCGCTCCTGTGGGTCCT (SEQ ID NO: 2) | CATTTTATGCACCAAAAGAGAAC TGCAATGTTTC (SEQ ID NO: 3) | 83 |
| HPV16-E6 v3 | GTTGAACCGAAACCGGTTA GT (SEQ ID NO: 7) | GTCGCTCCTGTGGGTCCT (SEQ ID NO: 2) | CATTTTATGCACCAAAAGAGAAC TGCAATGTTTC (SEQ ID NO: 3) | 84 |
| HPV16-E6 v4 | TTGAACCGAAACCGGTTAG TA (SEQ ID NO: 8) | GTCGCTCCTGTGGGTCCT (SEQ ID NO: 2) | CATTTTATGCACCAAAAGAGAAC TGCAATGTTTC (SEQ ID NO: 3) | 83 |
| HPV16-E6 v5 | CGAAACCGGTTAGTATAAA AGCA (SEQ ID NO: 9) | CTTTCTGGGTCGCTCCTGT (SEQ ID NO: 14) | CATTTTATGCACCAAAAGAGAAC TGCAATGTTTC (SEQ ID NO: 3) | 85 |
| HPV16-E6 v6 | TGAACCGAAACCGGTTAGT A (SEQ ID NO: 10) | GTCGCTCCTGTGGGTCCT (SEQ ID NO: 2) | CATTTTATGCACCAAAAGAGAAC TGCAATGTTTC (SEQ ID NO: 3) | 82 |
| HPV16-E6 v7 | CCGAAACCGGTTAGTATAA AAGC (SEQ ID NO: 11) | GTCGCTCCTGTGGGTCCT (SEQ ID NO: 2) | CATTTTATGCACCAAAAGAGAAC TGCAATGTTTC (SEQ ID NO: 3) | 78 |
| HPV16-E6 v8 | CGAAACCGGTTAGTATAAA AGCA (SEQ ID NO: 12) | GTCGCTCCTGTGGGTCCT (SEQ ID NO: 2) | CATTTTATGCACCAAAAGAGAAC TGCAATGTTTC (SEQ ID NO: 3) | 77 |
| HPV16-E6 v9 | CGAAACCGGTTAGTATAAA AGCAG (SEQ ID NO: 1) | GTCGCTCCTGTGGGTCCT (SEQ ID NO: 2) | CATTTTATGCACCAAAAGAGAAC TGCAATGTTTC (SEQ ID NO: 3) | 77 |

FIG .1D

FIG. 1E

FIG. 1F

FIG. 2A-2B

EP 4 352 258 B1

FIG. 2C

FIG. 2D

FIG. 2E

FIG. 3A

FIG. 3B-E

FIG. 4A-4B

FIG. 4C

FIG. 5A

FIG. 5B-D

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63208736

- WO 2020055834 A2

**Non-patent literature cited in the description**

- **HANNA et al.** Plasma HPV cell-free DNA monitoring in advanced HPV-associated oropharyngeal cancer. *Annals of Oncology*, 2018, vol. 29, 1980-1986
- **HINSON et al.** *Anal. Chem.*, 2011, vol. 83, 8604-8610
- **PINHEIRO et al.** *Anal. Chem.*, 2012, vol. 84, 1003-1011
- **CHATFIELD-REED K** ; **ROCHE VP** ; **PAN Q**. cfDNA detection for HPV+ squamous cell carcinomas. *Oral Oncol*, 2021, vol. 115, 104958, https://doi. org/10.1016/j.oraloncology.2020.104958
- **MACHTAY M** ; **MOUGHAN J** ; **TROTTI A** ; **GARDEN AS** ; **WEBER RS** ; **COOPER JS et al.** Factors Associated With Severe Late Toxicity After Concurrent Chemoradiation for Locally Advanced Head and Neck Cancer: An RTOG Analysis. *JCO*, 2008, vol. 26, 3582-9, https://doi.org/10.1200/JCO.2007.14.8841
- **CHERA BS** ; **AMDUR RJ** ; **GREEN R** ; **SHEN C** ; **GUPTA G** ; **TAN X et al.** Phase II Trial of De-Intensified Chemoradiotherapy for Human Papillomavirus-Associated Oropharyngeal Squamous Cell Carcinoma. *JCO*, 2019, vol. 37, 2661-9, https://doi. org/10.1200/JCO.19.01007
- **CHEN AM** ; **FELIX C** ; **WANG P-C** ; **HSU S** ; **BASEHART V** ; **GARST J et al.** Reduced-dose radiotherapy for human papillomavirus-associated squamous-cell carcinoma of the oropharynx: a single-arm, phase 2 study. *The Lancet Oncology*, 2017, vol. 18, 803-11, https://doi.org/10.1016/S1470-2045(17)30246-2
- **FERRIS RL** ; **FLAMAND Y** ; **WEINSTEIN GS** ; **LI S** ; **QUON H** ; **MEHRA R et al.** Transoral robotic surgical resection followed by randomization to low- or standard-dose IMRT in resectable p16+ locally advanced oropharynx cancer: A trial of the ECOG-ACRIN Cancer Research Group (E3311). *JCO*, 2020, vol. 38, 6500-6500, https://doi.org/10.1200/JCO.2020.38.15_suppl.6500

- **NICHOLS AC** ; **THEURER J** ; **PRISMAN E** ; **READ N** ; **BERTHELET E** ; **TRAN E et al.** Radiotherapy versus transoral robotic surgery and neck dissection for oropharyngeal squamous cell carcinoma (ORATOR): an open-label, phase 2, randomised trial. *The Lancet Oncology*, 2019, vol. 20, 1349-59, https://doi. org/10.1016/S1470-2045(19)30410-3
- **AHN SM** ; **CHAN JYK** ; **ZHANG Z** ; **WANG H** ; **KHAN Z** ; **BISHOP JA et al.** Saliva and plasma quantitative polymerase chain reaction-based detection and surveillance of human papillomavirus-related head and neck cancer. *JAMA Otolaryngol Head Neck Surg*, 2014, vol. 140, 846-54, https://doi. org/10.1001/jamaoto.2014.1338
- **DAHLSTROM KR** ; **LI G** ; **HUSSEY CS** ; **VO JT** ; **WEI Q** ; **ZHAO C et al.** Circulating human papillomavirus DNA as a marker for disease extent and recurrence among patients with oropharyngeal cancer. *Cancer*, 2015, vol. 121, 3455-64, https://doi. org/10.1002/cncr.29538
- **HANNA GJ** ; **SUPPLEE JG** ; **KUANG Y** ; **MAHMOOD U** ; **LAU CJ** ; **HADDAD RI et al.** Plasma HPV cell-free DNA monitoring in advanced HPV-associated oropharyngeal cancer. *Ann Oncol*, 2018, vol. 29, 1980-6, https://doi.org/10.1093/annonc/mdy251
- **DAMERLA RR** ; **LEE NY** ; **YOU D** ; **SONI R** ; **SHAH R** ; **REYNGOLD M et al.** Detection of Early Human Papillomavirus-Associated Cancers by Liquid Biopsy. *JCO Precis Oncol*, 2019, 3, https://doi. org/10.1200/PO.18.00276
- **CHERA BS** ; **KUMAR S** ; **BEATY BT** ; **MARRON D** ; **JEFFERYS S** ; **GREEN R et al.** Rapid Clearance Profile of Plasma Circulating Tumor HPV Type 16 DNA during Chemoradiotherapy Correlates with Disease Control in HPV-Associated Oropharyngeal Cancer. *Clin Cancer Res*, 2019, vol. 25, 4682-90, https://doi.org/10.1158/1078-0432.CCR-19-0211
- **CHERA BS** ; **KUMAR S** ; **SHEN C** ; **AMDUR R** ; **DAGAN R** ; **GREEN R et al.** Plasma Circulating Tumor HPV DNA for the Surveillance of Cancer Recurrence in HPV-Associated Oropharyngeal Cancer. *J Clin Oncol*, 2020, vol. 38, 1050-8, https://doi. org/10.1200/JCO.19.02444

- **AVANZINI S** ; **KURTZ DM** ; **CHABON JJ** ; **MODING EJ** ; **HORI SS** ; **GAMBHIR SS et al.** A mathematical model of ctDNA shedding predicts tumor detection size. *Sci Adv*, 2020, 6, https://doi.org/10.1126/sciadv. abc4308

- **HARING CT** ; **BHAMBANI C** ; **BRUMMEL C** ; **JEWELL B** ; **BELLILE E** ; **HEFT NEAL M et al.** Human Papilloma Virus Circulating Tumor DNA Assay Predicts Treatment Response in Recurrent/-Metastatic Head and Neck Squamous Cell Carcinoma. *Oncotarget*